(19) Europäisches Patentamt
European Patent Office
Office européen des brevets

(11) Veröffentlichungsnummer: **0 115 252**
**A2**

(12) # EUROPÄISCHE PATENTANMELDUNG

(21) Anmeldenummer: 83810620.1

(22) Anmeldetag: 23.12.83

(51) Int. Cl.³: A 61 K 7/06

(30) Priorität: 29.12.82 CH 7609/82

(43) Veröffentlichungstag der Anmeldung: 08.08.84 Patentblatt 84/32

(84) Benannte Vertragsstaaten: BE CH DE FR GB IT LI NL

(71) Anmelder: CIBA-GEIGY AG
Postfach
CH-4002 Basel(CH)

(72) Erfinder: Moldovanyi, Laszlo, Dr.
Austrasse 78
CH-4051 Basel(CH)

(72) Erfinder: Fearnley, Charles, Dr.
Wettsteinanlage 50
CH-4125 Riehen(CH)

(54) **Gemische aus quaternären, polymeren Ammoniumsalzen, Tensiden und Fettsäuren, deren Herstellung und Verwendung in kosmetischen Mitteln.**

(57) Wässrige Zusammensetzungen, die einen Ph-Wert von höchstens 7,1 aufweisen und in neuartiger Kombination mindestens

- quaternäre, polymere Ammoniumsalze (Homo- oder Copolymere mit Molekulargewichten von $10^3$ bis $10^9$)
- anionische oder nicht-ionische Tenside oder solche mit intramolekular einer positiven und einer oder zwei negativen Ladungen oder Gemische der Tenside der angegebenen Art und
- Fettsäuren

enthalten, sind als kosmetische Mittel verwendbar, vorausgesetzt, dass bei Anwesenheit von Homopolymeren nicht-ionische Tenside oder solche mit intramolekular je einer positiven und negativen Ladung eingesetzt werden und dass Tenside mit intramolekular einer positiven und einer oder zwei negativen Ladungen als Gemische mit anionischen und oder nicht-ionischen Tensiden vorliegen. Die Homo- oder Copolymere sind unter Ionenaustausch mit den Tensiden umgesetzt, sofern anionische Tenside oder solche mit intramolekular einer positiven und zwei negativen Ladungen eingesetzt werden. Bei der Applikation dieser kosmetischen Mittel auf Haar werden insbesondere beim Shampoonieren unter Entwicklung eines cremigen, stabilen Schaums ausgezeichnete Konditioniereffekte erzielt.



Croydon Printing Company Ltd

1-14255/+

Gemische aus quaternären, polymeren Ammoniumsalzen, Tensiden und Fettsäuren, deren Herstellung und Verwendung in kosmetischen Mitteln.

In der Haarpflege stellt die Reinigung der Haare einen der wichtigsten Vorgänge dar. Als Reinigungsmittel werden zweckmässig synthetische Waschrohstoffe eingesetzt, die den Vorteil aufweisen, auch bei hoher Wasserhärte ihre volle Reinigungskraft zu behalten. Bei alleiniger Verwendung von Waschrohstoffen zeigt das behandelte Haar in der Regel keine Konditioniereffekte.

Der vorliegenden Erfindung liegt nun die Aufgabe zugrunde, ein für kosmetische Zwecke, insbesondere für die Haarpflege geeignetes kostengünstiges Reinigungsmittel zur Verfügung zu stellen, das neben einer guten Waschwirkung die Erzielung ausgezeichneter Konditioniereffekte auf dem Haar ermöglicht. Diese sog. Konditioniereffekte stellen einen Sammelbegriff für u.a. folgende erwünschte Eigenschaften des behandelten Haares dar:

- leichte Nass- und Trockenkämmbarkeit
- Verhinderung der statischen Aufladung
- keine negative Beeinträchtigung des ursprünglichen Glanzes
- Aspekt, insbesondere Volumen und Fülle
- Griff.

Aus den europäischen Patentanmeldungen 45 720 und 47 714 sind Zusammensetzungen aus polymeren quaternären Ammoniumsalzen und anionischen oder nicht-ionischen Tensiden bekannt, mit welchen gute Konditioniereffekte erreichbar sind. Nun wurde gefunden, dass durch einen Zusatz von preisgünstigen Fettsäuren als ökologisch unbedenklichen Naturstoffen zu den bekannten Zusammensetzungen eine wesentliche Verbesserung der Konditioniereffekte erzielt werden kann. Gleichzeitig werden neben den erhöhten Konditioniereffekten unerwünschte Akkumuliereffekte auf dem behandelten Haar gemieden.

Durch die Kombination von Fettsäuren mit polymeren, quaternären Ammoniumsalzen in Gegenwart von Tensiden in neuartiger Zusammensetzung ist es erfindungsgemäss auf unerwartete Weise gelungen, Zusammensetzungen zur Verfügung zu stellen, die eine erhöhte Stabilität aufweisen und zu ausgezeichneten, verbesserten Konditioniereffekten führen.

Gegenstand der vorliegenden Erfindung ist somit eine wässrige Zusammensetzung aus polymeren, quaternären Ammoniumsalzen, Tensiden und Fettsäuren, die dadurch gekennzeichnet ist, dass sie einen pH-Wert von höchstens 7,1 aufweist und mindestens

(A) quaternäre Copolymerisate aus Vinylpyrrolidon, Estern der Acryl- oder Methacrylsäure und Alkylaminoalkanolen, Poly(dialkyl-alkenylen-ammoniumhalogenid)-$\alpha,\omega$-(trialkanolammoniumhalogenid)-Verbindungen, Copolymerisate aus Dialkyldialkenylenammoniumsalzen und Acrylamid oder Methacrylamid, Copolymerisate aus Acryl- oder Methacrylsäure, -amid oder -nitril und quaternären Ammoniumsalzen der Acrylsäurereihe, quaternäre Copolymerisate auf Basis von Polyamid-Aminen oder, sofern nicht-ionische Tenside oder solche mit intramolekular je einer positiven und negativen Ladung als Komponenten ($B_2$) und ($B_3$) mindestens teilweise eingesetzt werden, auch Homopolymerisate aus Dialkyldialkenylenammoniumsalzen, Homopolymerisate aus quaternären Ammoniumsalzen der Acrylsäurereihe oder Homopolymerisate aus Vinyl-N-alkyl- oder -N-alkenylpyridiniumsalzen enthält, wobei die Komponente (A) in

wässrigen Tensidsystemen löslich oder mikroemulgierbar ist und eine Molekulargewichtsverteilung von $10^3$ bis $10^9$ aufweist,

($B_1$) ein anionisches Tensid,

($B_2$) ein nicht-ionisches Tensid aus der Gruppe der Fettsäureester von 3- bis 6-wertigen Alkoholen oder von Mono- oder Disacchariden, der alkoxylierten Fettamine, Fettalkohole, Fettsäuren, Fettsäureamide, Alkylphenole oder Kohlenhydrate oder der Addukte aus Aethylen- und Propylenoxyd,

($B_3$) ein Tensid mit intramolekular je einer positiven und negativen Ladung

oder

($B_4$) ein Tensid mit intramolekular einer positiven und zwei negativen Ladungen

wobei die Tenside ($B_3$) und ($B_4$) als Gemisch mit mindestens einem der Tenside ($B_1$) und ($B_2$) eingesetzt werden, und

(C) eine Fettsäure mit 12 bis 22 Kohlenstoffatomen

enthält, wobei bei Einsatz eines anionischen Tensides und gegebenenfalls eines Tensides mit intramolekular einer positiven und zwei negativen Ladungen als Komponenten ($B_1$) und ($B_4$) das Tensid mindestens teilweise mit der Komponente (A) unter Ionenaustausch umgesetzt ist.

Tenside mit intramolekular je einer positiven und negativen Ladung als Komponente ($B_3$) werden auch zwitterionische Tenside genannt. Sie stellen ebenfalls amphotere Tenside dar, die am isolektrischen Punkt vorliegen. Tenside mit intramolekular einer positiven und zwei negativen Ladungen als Komponente ($B_4$) stellen anionische, zwitterionische oder amphotere Tenside dar.

In den erfindungsgemässen Zusammensetzungen werden die amphoteren Tenside ($B_3$) und ($B_4$) nie für sich allein, sondern nur stets im Gemisch mit anionischen Tensiden ($B_1$) und/oder nicht-ionischen Tensiden ($B_2$) eingesetzt. Somit enthalten die Zusammensetzungen die folgenden möglichen Kombinationen von Komponenten:

- (A), ($B_1$) und (C)
- (A), ($B_2$) und (C)
- (A), ($B_1$), ($B_2$) und (C)
- (A), ($B_1$), ($B_3$) und (C)
- (A), ($B_2$), ($B_3$) und (C)
- (A), ($B_1$), ($B_2$), ($B_3$) und (C)
- (A), ($B_1$), ($B_4$) und (C)
- (A), ($B_2$), ($B_4$) und (C)
- (A), ($B_1$), ($B_2$), ($B_4$) und (C) und
- (A), ($B_1$) ($B_2$), ($B_3$), ($B_4$) und (C)

Im Vordergrund des Intersses stehen Zusammensetzungen, welche die Komponenten (A), ($B_2$) und (C), vor allem (A), ($B_2$), ($B_4$) und (C) und insbesondere (A), ($B_1$) und (C) enthalten.

Die bei der Definition der Komponente (A) drei letztgenannten Homopolymerisate sind in nicht zwitterionischen, anionischen Tensiden als Komponente ($B_1$) nicht genügend löslich, um in den erfindungsgemässen Zusammensetzungen in Abwesenheit von nicht-ionischen Tensiden ($B_2$) oder deren Gemische mit amphoteren Tensiden ($B_3$) mit intramolekular je einer positiven und negativen Ladung eingesetzt zu werden. Bei Einsatz der genannten Homopolymerisate als Komponente (A) muss somit die Zusammensetzung nicht-ionische Tenside als Komponente ($B_2$) und gegebenenfalls Tenside mit intramolekular je einer positiven und negativen Ladung als Komponente ($B_3$) oder deren Gemische mit nicht zwitterionischen anionischen Tensiden als Komponente ($B_1$) und gegebenenfalls Tensiden mit intramolekular einer positiven und zwei negativen Ladungen als Komponente ($B_4$) enthalten.

Copolymerisate der vorstehend angegebenen Art sind den drei letztgenannten Homopolymerisaten gegenüber als Komponente (A) in den erfindungsgemässen Zusammensetzungen bevorzugt, da sie auch in Gegenwart von nicht zwitterionischen, anionischen Tensiden für sich allein als Komponente ($B_1$) oder deren Gemische mit Tensiden mit intramolekular einer positiven und zwei negativen Ladungen als Komponente ($B_4$) zu Zusammensetzungen aus (A), ($B_1$) und (C) oder (A), ($B_1$), ($B_4$) und (C) führen, die z.B. zu stabilen Shampoos fomulierbar sind.

Bevorzugte Copolymerisate aus Vinylpyrrolidon und Acrylaten oder Methacrylaten als Komponente (A) weisen ein Molekulargewicht von $1,5 \cdot 10^4$ bis $1 \cdot 10^6$, in beliebiger Reihenfolge durchschnittlich 20 bis 99 Mol% wiederkehrende Strukturelemente der Formel

(1) 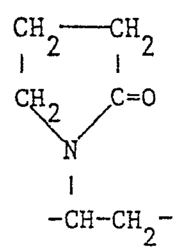

und durchschnittlich 1 bis 80 Mol% wiederkehrende Strukturelemente der Formel

(2) 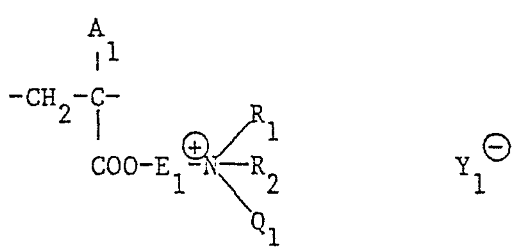

auf, worin $A_1$ Wasserstoff oder Methyl, $E_1$ gegebenenfalls durch Hydroxyl substituiertes Alkylen mit 2 bis 18 Kohlenstoffatomen, $R_1$ und $R_2$ je Alkyl mit 1 bis 4 Kohlenstoffatomen, $Q_1$ Wasserstoff, Methyl, Aethyl oder Benzyl und $Y_1^{\ominus}$ ein Sulfat- oder Halogenidanion, ein Alkylsulfat- oder Alkylphosphation oder das Anion einer Alkylcarbonsäure mit je 1 bis 4 Kohlenstoffatomen im Alkylrest bedeuten. Copolymerisate dieser Art sind z.B. in der französischen Patentschrift 2,077,143 beschrieben. Bevorzugte Copolymerisate weisen durchschnittlich 20 bis 90 Mol % wiederkehrende Strukturelemente der Formel (1) und durchschnittlich 10 bis 80 Mol % wiederkehrende Strukturelemente der Formel (2) auf, worin $E_1$ vorzugsweise Aethylen oder Propylen und $R_1$ und $R_2$ vorzugsweise je Methyl oder Aethyl bedeuten.

Bevorzugte Poly(dialkylalkenylenammoniumhalogenid)-$\alpha,\omega$-(trialkanolammoniumhalogenid)-Verbindungen als Komponente (A) sind z.B. Poly(dimethylbutenylenammoniumchlorid)-$\alpha,\omega$-bis(triäthanolammoniumchlorid)-Verbindungen, die ein Molekulargewicht von 1000 bis 5000 aufweisen und der Formel

(3) 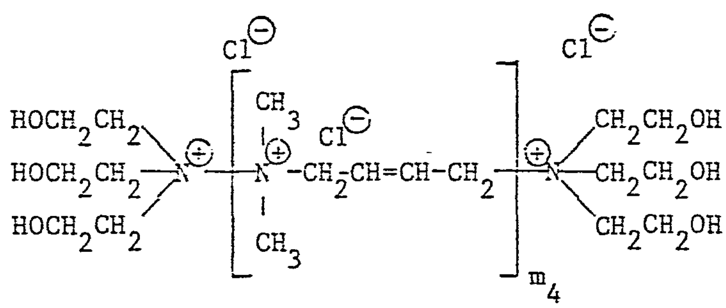

entsprechen, worin $m_4$ eine ganze Zahl von 4 bis 35 ist.

Bevorzugte Copolymerisate aus Dialkyldialkenylenammoniumsalzen und Acrylamid oder Methacrylamid als Komponente (A) sind z.B. Copolymerisate aus Dimethyldiallylammoniumchlorid und Acrylamid, die ein Molekulargewicht von $5\cdot10^3$ bis $5\cdot10^6$, vorzugsweise $10^4$ bis $3\cdot10^6$ und in beliebiger Reihenfolge durchschnittlich 20 bis 99 Mol% wiederkehrende Strukturelemente der Formel

(4) 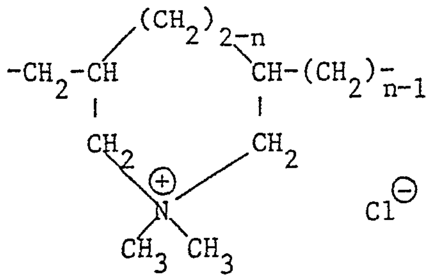 ,

worin n 1 oder 2 ist, und durchschnittlich 1 bis 80 Mol% wiederkehrende Strukturelemente der Formel

(5) 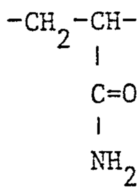

aufweisen. Bevorzugt sind Copolymerisate eines Molekulargewichtes von $10^4$ bis $3 \cdot 10^6$, welche in beliebiger Reihenfolge durchschnittlich 20 bis 90 Mol% wiederkehrende Strukturelemente der Formel (4), worin n 2 ist, und durchschnittlich 10 bis 80 Mol% wiederkehrende Strukturelemente der Formel (5) aufweisen.

Verbindungen der Formel (3) und Copolymerisate mit wiederkehrenden Strukturelementen der Formeln (4) und (5) sind z.B. in der deutschen Offenlegungsschrift 3 029 306 beschrieben.

Bevorzugte Copolymerisate von Verbindungen der Acrylsäurereihe und quaternären Ammoniumsalzen der Acrylsäurereihe als Komponente (A) weisen eine Molekulargewichtsverteilung von $10^4$ bis $10^9$ und in beliebiger Reihenfolge wiederkehrende Strukturelemente der Formel

(6) 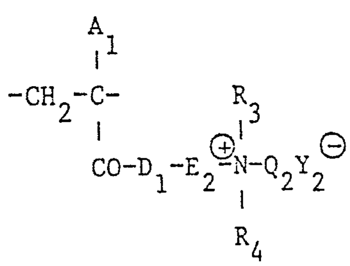 ,

wiederkehrende Strukturelemente der Formel

(7) 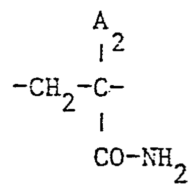

und gegebenenfalls wiederkehrende Strukturelemente der Formeln

(8) 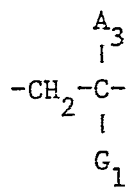 und (9) 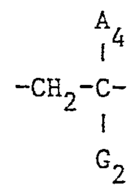

auf, worin $A_1$, $A_2$, $A_3$ und $A_4$ je Wasserstoff oder Methyl, $G_1$ und $G_2$ voneinander verschieden sind und je -CN, -COOH oder

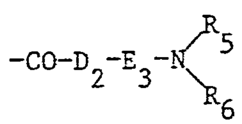 , $D_1$ und $D_2$ je Sauerstoff oder -NH-, $E_2$ und $E_3$ je Alkylen mit 1 bis 4 Kohlenstoffatomen, das gegebenenfalls durch Hydroxyl substituiert ist, $R_3$, $R_4$, $R_5$ und $R_6$ je Methyl oder Aethyl, $Q_2$ Alkyl, Hydroxyalkyl mit 1 bis 4 Kohlenstoffatomen oder Benzyl und $Y_2^{\ominus}$ ein Sulfat- oder Halogenidanion oder ein Alkylsulfat- oder Alkylphosphation mit 1 bis 4 Kohlenstoffatomen im Alkylrest bedeuten.

Die Copolymerisate der angegebenen Art enthalten Strukturelemente der Formeln (6) und (7), der Formeln (6), (7) und (8) oder der Formeln (6), (7), (8) und (9).

In einer bevorzugten Ausführungsform enthalten die Copolymerisate Strukturelemente der Formeln (6), (7) und gegebenenfalls (8) und (9) und weisen eine Molekulargewichtsverteilung von $10^4$ bis $10^9$ auf, wobei das Molekulargewicht von mindestens 5 Gewichtsprozent des copolymeren Ammoniumsalzes $10^7$ bis $10^9$ beträgt. Solche Copolymerisate zeichnen sich dadurch aus, dass sie vorzugsweise durch Wasser-in-Oel Emulsionspolymerisation oder Lösungspolymerisation eines quaternären Ammoniumsalzes der Acrylsäurereihe und mindestens eines weiteren Comonomers auf Acrylbasis erhältlich sind.

Durch Wasser-in-Oel Emulsionspolymerisation, auch inverse Emulsionspolymerisation genannt, oder durch Lösungspolymerisation, erreicht man den hohen Molekulargewichtsbereich der Copolymerisate von $10^7$ bis $10^9$, deren Anteil mindestens 5, vorzugsweise 10 bis 60 Gewichtsprozent der Copolymerisate innerhalb der breiten Molekulargewichts verteilung von $10^4$ bis $10^9$ ausmacht. Eine Anreicherung an Anteile eines Molekulargewichtes von $10^7$ bis $10^9$ innerhalb der breiten Molekulargewichtsverteilung von $10^4$ bis $10^9$ kann nötigenfalls auch durch Behandlung des Copolymers in einem vorzugsweise mit Wasser mischbaren Lösungsmittel, z.B. Methanol, Isopropanol oder Aceton, erzielt werden. Eine solche Behandlung wird vor allem nach durchgeführter Lösungspolymerisation durchgeführt. Besonders bevorzugte Polymere weisen 12 bis 50, insbesondere 35 bis 45 Gewichtsprozent Anteile im Molgewichtsbereich $10^7$ bis $10^9$ und weniger als 15 Gewichtsprozent Anteile im Molgewichtsbreich kleiner als $10^5$ auf.

Der Anteil an Strukturelementen der Formel (6) in den Copolymerisaten, auch Quatgehalt genannt, bildet neben der Molekulargewichtsverteilung ein weiteres, wesentliches Kennzeichen der eingesetzten Ammoniumsalze, welche in einer bevorzugten Ausführungsform durchschnittlich 1 bis 90 Mol % Strukturelemente der Formel (6), durchschnittlich 10 bis 99 Mol % Strukturelemente der Formel (7) und jeweils 0 bis durchschnittlich 10 Mol % Strukturelemente der Formel (8) und gegebenenfalls (9), d.h. (8) und/oder (9).

Copolymerisate, die Strukturelemente der Formeln (6), (7), (8) und (9) sind gegenüber Copolymerisaten, die Strukturelemente der Formeln (6), (7) und (8) aufweisen, bevorzugt. Im Vordergrund des Interesses stehen jedoch Copolymerisate, die nur die Strukturelemente der Formeln (6) und (7) aufweisen, wobei im allgemeinen durchschnittlich 1 bis 90, vorzugsweise 15 bis 80, insbesondere 20 bis 35 Mol % wiederkehrende Strukturelemente der Formel (6) und im allgemeinen durchschnittlich 10 bis 99, vorzugsweise 20 bis 85, insbesondere 65 bis 80 Mol % wiederkehrende Strukturelemente der Formel (7) vorhanden sind.

Als bevorzugte Bedeutungen für die Symbole der Formel (6) gelten für $A_1$ Methyl, für $D_1$ Sauerstoff, für $E_2$ unsubstituiertes n-Propylen oder insbesondere unsubstituiertes Aethylen, für $R_3$ und $R_4$ Methyl und für $Q_2$ unsubstituiertes Propyl, vorzugsweise Aethyl oder insbesondere Methyl. $A_2$ in Formel (7) steht vorzugsweise für Wasserstoff. Falls Strukturelemente der Formel (8) für sich allein, d.h. in Abwesenheit von Strukturelementen der Formel (9) vorhanden sind, gelten als bevorzugte Bedeutungen für die Symbole in Formel (8)

Methyl für $A_3$ und $-CO-D_2-E_3-N{<}^{R_5}_{R_6}$ für $G_1$, wobei $D_2$, $E_3$, $R_5$ und $R_6$ die gleichen bevorzugten Bedeutungen haben wie für $D_1$, $E_2$, $R_3$ und $R_4$ vorstehend angegeben. Falls Strukturelemente der Formel (9) zusätzlich zu den Strukturelementen der Formel (8) vorhanden sind, bedeuten in Formel (9) $A_4$ vorzugsweise Wasserstoff und $G_2$ vorzugsweise -CN oder $A_4$ insbesondere Methyl und $G_2$ insbesondere -COOH.

Somit sind Copolymerisate besonders bevorzugt, deren Molekulargewichtsverteilung $10^4$ bis $10^9$ beträgt, wobei etwa 10 bis 60 Gewichtsprozent der Copolymerisate ein Molekulargewicht von $10^7$ bis $10^9$ haben, und welche in beliebiger Reihenfolge durchschnittlich 1 bis 90 Mol % wiederkehrende Strukturelemente der Formel (6) und durchschnittlich 10 bis 99 Mol % wiederkehrende Strukturelemente der Formel (7) aufweisen und vor allem Copolymerisate, deren Molekulargewichtsverteilung $10^4$ bis $10^9$ beträgt, wobei 12 bis 50 Gewichtsprozent der Copolymerisate ein Molekulargewicht von $10^7$ bis $10^9$ hat, und welche in beliebiger Reihenfolge durchschnittlich 20 bis 35 Mol % wiederkehrende Strukturelemente der Formel

(10) 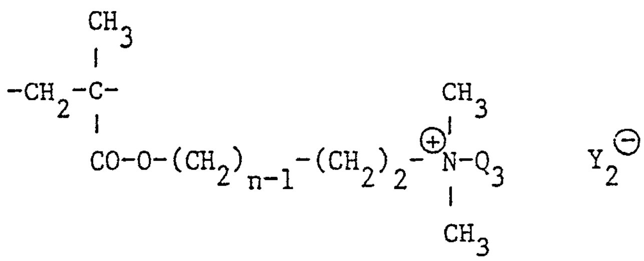 ,

worin n 1 oder 2 und $Q_3$ Propyl, Aethyl oder Methyl bedeuten und $Y_2^{\ominus}$ die angegebenen Bedeutungen hat,
und durchschnittlich 65 bis 80 Mol % wiederkehrende Strukturelemente der Formel (5) aufweisen.

Bevorzugte Copolymerisate auf Basis von Polyamid-Aminen weisen Molekulargewichte von $10^3$ bis $10^5$, vorzugsweise $10^3$ bis $10^4$ auf und sind z.B. aus aliphatischen, gesättigten Dicarbonsäuren mit 2 bis 10, vorzugsweise 3 bis 6 Kohlenstoffatomen, insbesondere Adipinsäure, und Polyalkylenpolyaminen, z.B. Polypropylen- und Polyäthylenpolyamin, erhältlich. Sie weisen in einer bevorzugten Ausführungsform in beliebiger Reihenfolge wiederkehrende Strukturelemente auf, die in wässrigen Lösungen eines pH-Wertes von höchstens 7,1 der Formeln

(11) $-CO-(CH_2)_4-CO-$ und

(12) 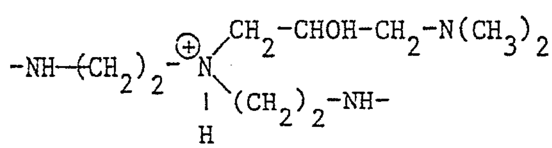

entsprechen, oder insbesondere wiederkehrende Strukturelemente, die bei den angegebenen Bedingungen der Formel

(13) 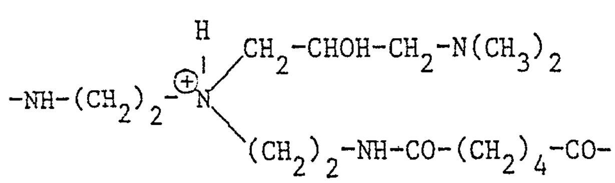

entsprechen.

Solche Copolymerisate sind z.B. in CTFA Cosmetic Ingredient Dictionary, 3. Auflage 1982, der Cosmetic, Toiletry and Fragrance Association beschrieben.

Falls in Gegenwart von nicht-ionischen Tensiden als Komponente ($B_2$) oder deren Gemische mit Tensiden mit intramolekularer je einer positiven und negativen Ladung als Komponente ($B_3$) Homopolymerisate aus Dialkyldialkenylenammoniumsalzen, aus quaternären Ammoniumsalzen der Acrylsäurereihe oder aus Vinyl-N-alkyl- oder -N-Alkenylpyridiniumsalzen als Komponente (A) eingesetzt werden, so handelt es sich vorzugsweise um Dimethyldiallylammoniumchloride, die ein Molekulargewicht von 4000 bis 550 000 und wiederkehrende Strukturelemente der Formel (4) aufweisen und die ebenfalls in der deutschen Offenlegungsschrift 3 029 306 beschrieben sind, um quaternäre Ammoniumsalze, die eine Molekulargewichtsverteilung von $10^4$ bis $10^9$ und wiederkehrende Strukturelemente der Formel (6) aufweisen, und um polymerisierte Vinylpyridiniumsalze, die ein Molekulargewicht von $5 \cdot 10^3$ bis $1 \cdot 10^5$ und wiederkehrende Strukturelemente der Formel

(14)

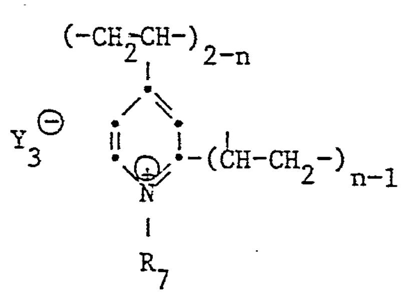

aufweisen, worin n 1 oder 2, $R_7$ Alkyl mit 1 bis 22 Kohlenstoffatomen oder Alkenyl mit 3 oder 4 Kohlenstoffatomen und $Y_3^{\ominus}$ ein Halogenid-oder Sulfat-Anion oder das Anion einer Alkylcarbonsäure mit 1 bis 4 Kohlenstoffatomen im Alkylrest bedeuten. Homopolymerisate mit wiederkehrenden Strukturelementen der Formel (14) sind ebenfalls in der deutschen Offenlegungsschrift 3 029 306 offenbart.

Quaternäre Ammoniumsalze mit wiederkehrenden Strukturelementen der Formel (6) stellen Homopolymeresate dar, in welchen der Quatgehalt, d.h. der Gehalt an Strukturelemente der Formel (6), 100 % beträgt. Der Quatgehalt von 100 % ist aber als Idealwert anzusehen, da durch Hydrolyse der Strukturelemente der Formel (6) die Homopolymerisate stets Spuren (z.B. etwa 0,01 bis 0,5 Gewichtsprozent) an Strukturelementen der Formel (9), worin $G_2$ -COOH bedeutet, enthalten.

Im Vordergrund des Interesses stehen jedoch als polymere Ammoniumsalze für die Komponente (A) der erfindungsgemässen Zusammensetzung Copolymerisate, die wiederkehrende Strukturelemente der Formel (3), oder der Formeln (4) und (5), vor allem der Formeln (11) und (12) oder insbesondere der Formeln (6), (7) und gegebenenfalls (8) und (9) aufweisen.

Anionische Tenside, die als Komponente ($B_1$) in den erfindungsgemässen Zusammensetzungen enthalten sind, entsprechen der Formel

(15) $$X_1^{\ominus} Z^{\oplus} ,$$

worin $Z^{\oplus}$ ein Alkalimetall-, insbesondere Natrium- oder ein Ammoniumkation bedeutet, wobei das Ammoniumkation ($NH_4^{\oplus}$) unsubstituiert oder vorzugsweise durch 1, 2 oder vor allem 3 Alkyl- oder Alkanolreste mit je 1 bis 4 Kohlenstoffatomen substituiert ist und $X_1^{\ominus}$ für den Rest eines anionischen Tensids steht. Anionische Tenside ($B_1$) der Formel (15) oder deren Gemische mit Tensiden ($B_4$) mit intramolekular einer positiven und zwei negativen Ladungen sind den nicht-ionischen Tensiden ($B_2$) oder deren Gemische mit Tensiden ($B_3$) mit intramolekular je einer positiven und negativen Ladung gegenüber bevorzugt.

$X_1^{\ominus}$ in Formel (15) steht für den Rest eines anionischen, nicht zwitterionischen Tensids. Hierbei, handelt es sich vorzugsweise um Reste oberflächenaktiver Sarkosinate, Sulfate, z.B. Alkylsulfate, Alkyläthersulfate, Alkylamidsulfate, Alkylamidäthersulfate, Alkylarylpolyäthersulfate oder Monoglyceridsulfate, Sulfonate, z.B. Alkylsulfonate, Alkylamidsulfonate, Alkylarylsulfonate, α-Oelfinsulfonate oder Sulfobernsteinsäurederivate, z.B. Alkylsulfosuccinate, Alkyläthersulfosuccinate, Alkylamidsulfosuccinate, Alkylamidpolyäthersulfosuccinate oder Alkylsulfosuccinamide, ferner auch um Reste von fluorierten Tensiden oder Phosphattensiden, wie z.B. Alkyl- oder Alkylätherphosphaten.

In Frage kommen z.B. für Sarkosinat-Tenside solche der Formel

(16) 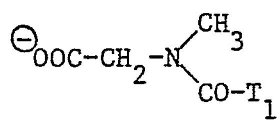 $Z^{\oplus}$ ,

worin $T_1$ Alkyl oder Alkenyl mit 7 bis 21, vorzugsweise 11 bis 17 Kohlenstoffatomen und $Z^{\oplus}$ die angegebenen Bedeutungen haben.

In Formel (16) leiten sich die Reste $T_1CO-$ von den entsprechenden gesättigten oder ungesättigten Fettsäuren mit 8 bis 22, vorzugsweise 12 bis 18 Kohlenstoffatomen ab. Als Beispiele der entsprechenden Fettsäuren seien Capryl-, Caprin-, Arachin- und Behensäure, insbesondere Laurin-, Myristin-, Palmitin- und Stearinsäure oder Myristolein-, Palmitolein-, Elaeostearin-, Clupandonsäure, insbesondere Oel-, Elaidin-, Eruka-, Linol- und Linolensäure genannt. Alkyl- und Alkenylreste für $T_1$ die sich von technischen Gemischen der genannten gesättigten und/oder ungesättigten Fettsäuren ableiten, sind besonders bevorzugt. Als spezifische Vertreter von Sarkosinat-Tensiden seien vor allem solche der Formeln

(17) 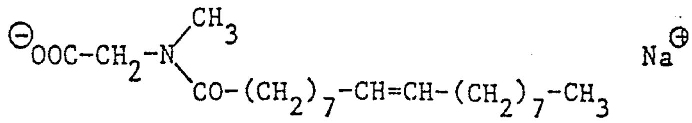

und insbesondere

(18) 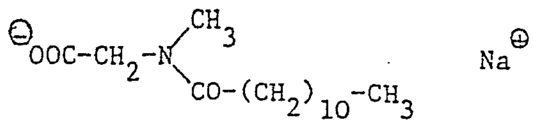

genannt.

Falls $X_1^{\ominus}$ einen Sulfatrest (von Alkylsulfaten, sowie von Aether-, Ester-, Amid- oder Aminosulfaten) bedeutet, kommen z.B. insbesondere Tenside der Formeln

(19) $$T_1-O-(CH_2-CH_2-O)_{p-1}-SO_3^{\ominus}Z^{\oplus},$$

(20) 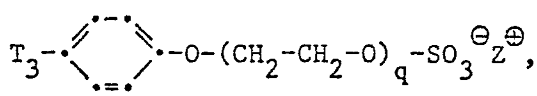

(21) $$T_1-CO-NH-(CH_2)_r-O-SO_3^{\ominus}Z^{\oplus}$$ oder

(22) $$T_1-CO-NH-(CH_2-CH_2-O)_p-SO_3^{\ominus}Z^{\oplus}$$

in Betracht, worin $T_3$ Alkyl oder Alkenyl mit 6 bis 14, vorzugsweise 8 bis 12 Kohlenstoffatomen, p eine ganze Zahl von 1 bis 50, vorzugsweise 1 bis 20, q eine ganze Zahl von 6 bis 12 und r eine ganze Zahl von 2 bis 6 bedeuten und $T_1$ und $Z^{\oplus}$ die angegebenen Bedeutungen haben.

Bevorzugte Alkylreste für $T_3$ in Formel (20) sind z.B. Alkylreste mit 6 bis 12 Kohlenstoffatomen wie n-Hexyl, i-Hexyl, n-Heptyl, n-Octyl, i-Octyl, n-Nonyl, i-Nonyl, n-Decyl, n-Dodecyl, ferner Alkylreste mit

12 bis 14 Kohlenstoffatomen, wie Lauryl und Myristyl, Alkenylreste wie Oleyl und Reste, die sich von dimerisierten Olefinen mit je 6 oder 7 Kohlenstoffatomen ableiten.

Als spezifische Vertreter von Sulfat-Tensiden seien vor allem solche der Formeln

(23) $^{\ominus}O_3S\text{-}O\text{-}(CH_2)_{2-4}\text{-}NH\text{-}CO\text{-}(CH_2)_{11}\text{-}CH_3\ Na^{\oplus}$,

(24) $^{\ominus}O_3S\text{-}(O\text{-}CH_2\text{-}CH_2)_{8-10}\text{-}NH\text{-}CO\text{-}(CH_2)_{11}\text{-}CH_3\ Na^{\oplus}$ ,

(25) 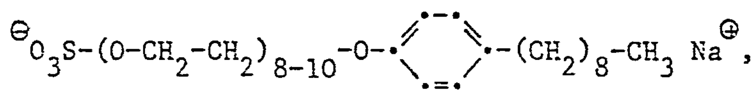,

und insbesondere

(26) $^{\ominus}O_3S\text{-}(O\text{-}CH_2\text{-}CH_2)_2\text{-}O\text{-}(CH_2)_{11}\text{-}CH_3\ Na^{\oplus}$ und

(27) 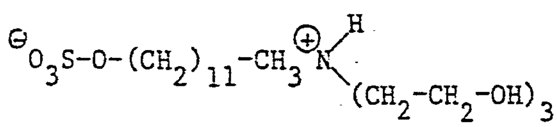

genannt.

Ist $X_1^{\ominus}$ ein Sulfonatrest, so kommen Tenside der Formeln

(28) 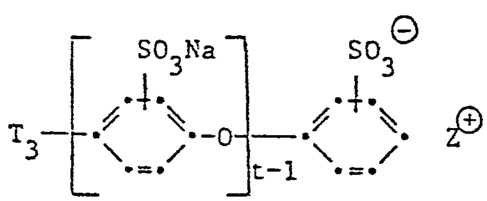 ,

(29) 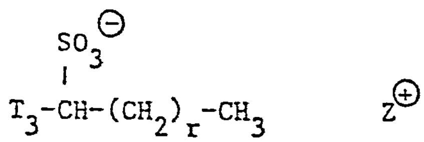 ,

(30) $T_1\text{-}CH_2\text{-}SO_3^{\ominus}\ Z^{\oplus}$ ,

(31) $T_3\text{-COO-}CH_2\text{-}CH_2\text{-}SO_3^{\ominus}\ Z^{\oplus}$ ,

(32) $T_3\text{-CO-}\overset{\displaystyle CH_3}{\overset{|}{N}}\text{-}CH_2\text{-}CH_2\text{-}SO_3^{\ominus}\ Z^{\oplus}$ ,

(33) $T_1\text{-}CH_2\text{-}\overset{\displaystyle SO_3}{\overset{|}{C}}H\text{-}T_1'\ Z^{\oplus}$ ,

(34) $T_1\text{-CH=CH-}SO_3^{\ominus}\ Z^{\oplus}$ oder

(35) $T_1\text{-}\overset{\displaystyle OH}{\overset{|}{C}}H\text{-}CH_2\text{-}SO_3^{\ominus}\ Z^{\oplus}$

in Betracht, worin $T_1$, $T_3$, $Z^{\oplus}$ und r die angegebenen Bedeutungen haben, $T_1'$ die für $T_1$ angegebenen Bedeutungen hat, wobei $T_1$ und $T_1'$ voneinander verschieden oder vorzugsweise gleich sind und t 1 oder 2 bedeutet.

Als spezifische Vertreter solcher Sulfonat-Tenside seien vor allem die Reste der Formeln

(36) $CH_3\text{-}(CH_2)_{11}\text{-}\overset{\displaystyle SO_3^{\ominus}}{\overset{|}{C}}H\text{-}(CH_2)_{2\text{-}4}\text{-}CH_3\ Na^{\oplus}$ ,

(37) $CH_3\text{-}(CH_2)_{12\text{-}22}\text{-}CH_2\text{-}SO_3^{\ominus}\ Na^{\oplus}$ ,

(38) $^{\ominus}O_3S\text{-}(CH_2)_2\text{-OOC-}(CH_2)_{10}\text{-}CH_3\ NH_4^{\oplus}$ ,

(39) $^{\ominus}O_3S\text{-}(CH_2)_2\text{-}N\begin{matrix}\diagup CH_3\\ \diagdown CO\text{-}(CH_2)_7\text{-CH=CH-}(CH_2)_7\text{-}CH_3\end{matrix}\ Na^{\oplus}$ ,

und insbesondere

(40) 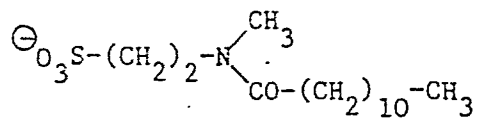 und

(41) 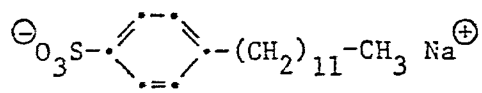

erwähnt.

Bedeutet $X_1^{\ominus}$ den Rest eines Sulfobernsteinsäurederivats, so entspricht das Tensid z.B. einer der Formeln

(42) 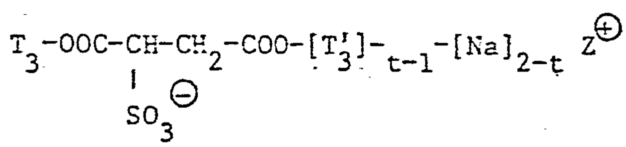 ,

(43) 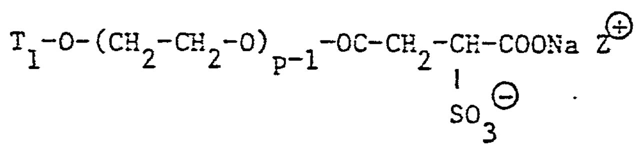 ,

(44) 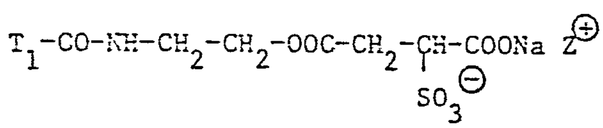 ,

(45) 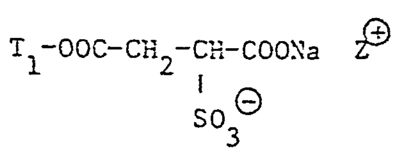 ,

(46) 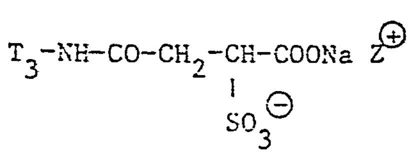 ,

(47) 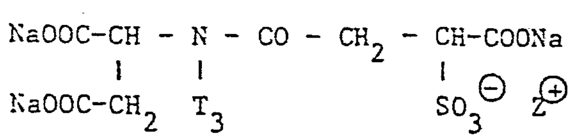 oder

(48) 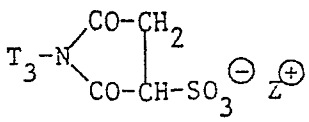 ,

worin $T_1$, $T_3$, $Z^{\oplus}$, p und t die angegebenen Bedeutungen haben und $T_3'$ die für $T_3$ angegebenen Bedeutungen hat, wobei $T_3$ und $T_3'$ voneinander verschieden oder vorzugsweise gleich sind.

Als spezifische Vertreter solcher Tenside seinen die Sulfobernsteinsäurederivate der Formeln

(49) 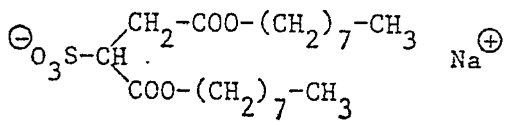 ,

(50) 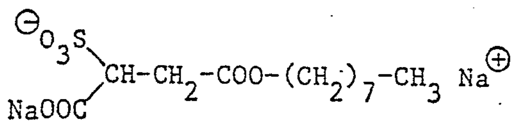 ,

(51) 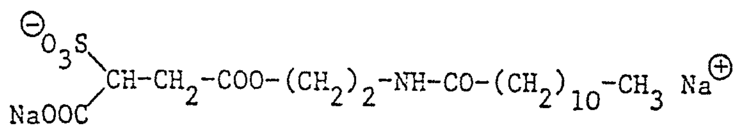 ,

(52) 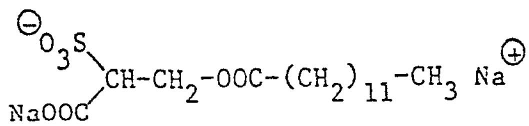 ,

(53) 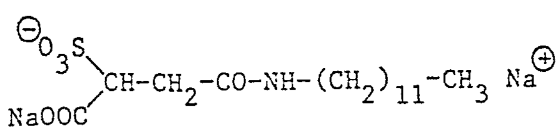 ,

(54) 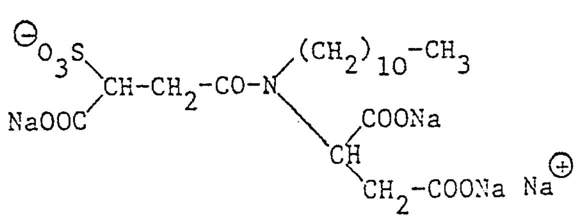 ,

(55) 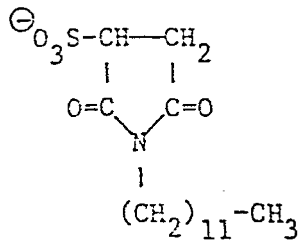 $Na^{\oplus}$ und insbesondere

(56) 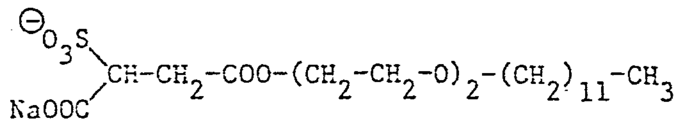 $Na^{\oplus}$

erwähnt.

Als mögliches fluoriertes Tensid kommen z.B. Tenside der Formel

(57) 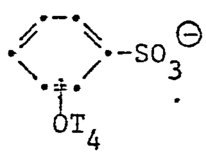 $Z^{\oplus}$

in Frage, worin $T_4$ perfluoriertes Alkyl oder Alkenyl mit 6 bis 14 Kohlenstoffatomen bedeutet und $Z^{\oplus}$ die angegebenen Bedeutungen hat.

Perfluorierte Tenside der Formeln

(58) $C_{10}F_{19}OC_6H_4SO_3^{\ominus}$ $Na^{\oplus}$

oder

(59) $C_{10}F_{21}OC_6H_4SO_3^{\ominus}$ $Na^{\oplus}$

stellen spezifische Beispiele solcher Tenside dar.

Als Phosphattenside kommen vor allem solche der Formeln

(60) 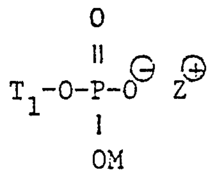 ,

(61) 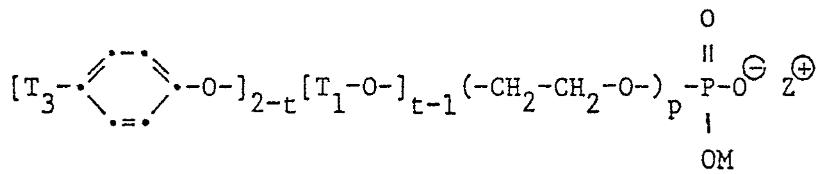

oder

(62) 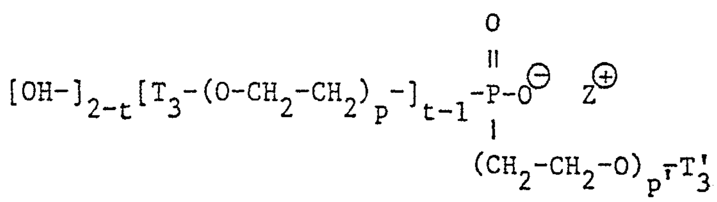

in Frage, worin M Wasserstoff, Ammonium, ein Alkalimetall oder Alkyl mit 1, 2 oder 3 Kohlenstoffatomen ist, $T_1$, $T_3$, $T'_3$, $Z^{\oplus}$, p und t die angegebenen Bedeutungen haben, p' die für p angegebene Bedeutung hat und p und p' voneinander verschieden oder vorzugsweise gleich sind.

Als Beispiele solcher Phosphattenside seien solche der Formeln

(63) 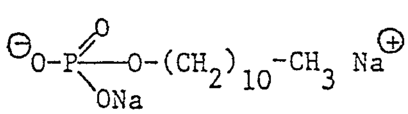 ,

(64) 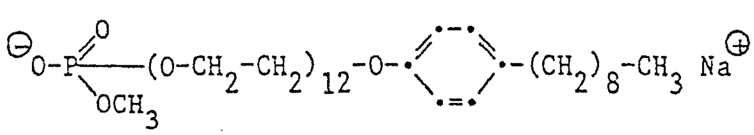 ,

(65) 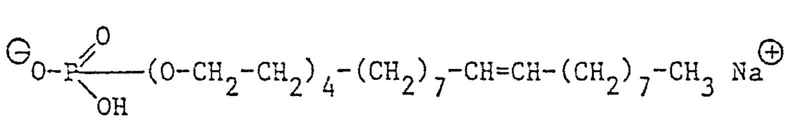 ,

(66) 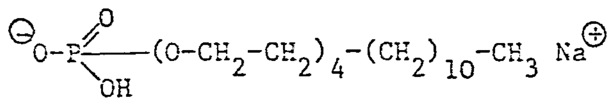,

(67) 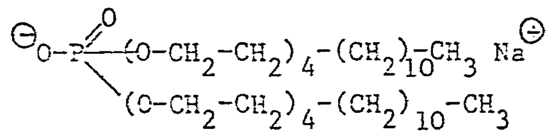,

(68) 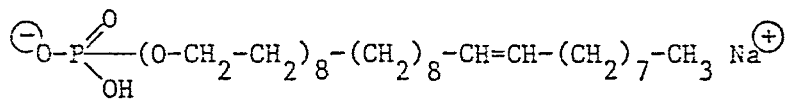,

(69) 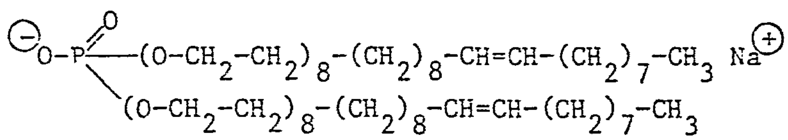,

(70) 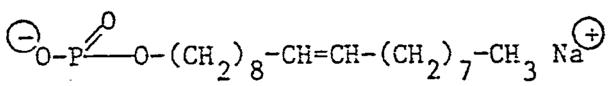,

(71) 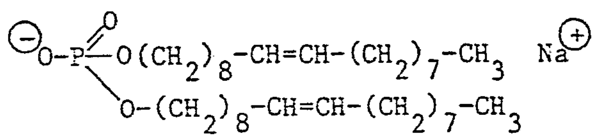

und deren Gemische, z.B. technische Gemische von Phosphattensidresten der Formeln (66) und (67), der Formeln (68) und (69) oder der Formeln (70) und (71) genannt.

Als nicht-ionische Tenside für die Komponente ($B_2$) in den erfindungsgemässen Zusammensetzungen kommen alkoxylierte, vorzugsweise propoxylierte und insbesondere äthoxylierte Fettamine, vor allem aber äthoxylierte Fettalkohole, Fettsäuren, Fettsäureamide, Alkylphenole oder Kohlenhydrate, worin die endständigen Hydroxylgruppen gegebenenfalls veräthert sind, und insbesondere als Alkyläther mit 1 bis 20 Kohlenstoffatomen im Aetherteil vorliegen oder insbesondere Addukte (Blockcopolymere) aus Aethylen- und Propylenoxyd in Betracht.

Geeignet sind ferner auch Fettsäureester von 3- bis 6-wertigen Alkoholen (Glycerin, Pentaerythrit, Sorbit, Sorbitan) oder von Mono- oder Disacchariden (Saccharose).

Die als nicht-ionische Tenside verwendeten, äthoxylierten Fettalkohole, Fettsäuren, Fettsäureamide, Alkylphenole oder Kohlenhydrate entsprechen vorzugsweise einer der Formeln

(72) $H-(O-CH_2-CH_2)_p-OOC-T_1$ ,

(73) $H-(O-CH_2-CH_2)_p-NH-CO-T_1$ ,

(74) $H-(O-CH_2-CH_2)_p-O-T_2$ ,

(75) 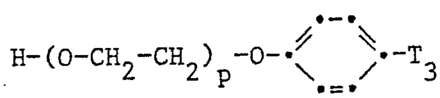 oder

(76) $H-(O-CH_2-CH_2)_p-O-CH_2-(CHOH)_s-CHO$ ,

worin $T_1$ Alkyl oder Alkenyl mit 7 bis 21, vorzugsweise 11 bis 17 Kohlenstoffatomen, $T_2$ Alkyl oder Alkenyl mit 8 bis 22, vorzugsweise 12 bis 18 Kohlenstoffatomen, $T_3$ Alkyl oder Alkenyl mit 6 bis 14, vorzugsweise 8 bis 12 Kohlenstoffatomen, p eine ganze Zahl von 1 bis 50, vorzugsweise 1 bis 20, und s 3 oder vorzugsweise 4 bedeuten.

In Formel (72) leiten sich die Reste $T_1COO-$ von den entsprechenden gesättigten oder ungesättigten Fettsäuren mit 8 bis 22, vorzugsweise 12 bis 18 Kohlenstoffatomen ab. Hierbei handelt es sich im allgemeinen um die bei der Definition des Restes $T_1-CO-$ in Formel (16) vorstehend erwähnten Fettsäuren bzw. Fettsäuregemische.

Die Fettsäureamid- und Fettalkoholreste $T_1$-CO-NH- und $T_2$-O- in den Formeln (73) und (74) leiten sich vorzugsweise ebenfalls von den entsprechenden erwähnten Fettsäuren ab.

Bevorzugte Alkylreste für $T_3$ in Formel (75) sind bei den bevorzugten Definitionen von $T_3$ in Formel (2) vorstehend angegeben.

Als äthoxylierte Kohlenhydrate kommen je nach dem Wert von s in Formel (76) vor allem äthoxylierte Pentosen und insbesondere Hexosen, z.B. äthoxylierte Glukose, in Betracht.

Gegebenenfalls können die äthoxylierten Tenside der Formel (72) bis (76) auch in verätherter Form (Alkyläther mit 1 bis 20 Kohlenstoffatomen im Aetherteil) vorliegen.

Unter den äthoxylierten, nicht-ionischen Tensiden der Formeln (72) bis (76) sind die äthoxylierten Fettsäuren und Fettalkohole der Formeln (72) und (74) und insbesondere die äthoxylierten Alkylphenole der Formel (75) bevorzugt. Als spezifische Beispiele solcher äthoxylierter Alkylphenole sei u.a. das Tensid der Formel

(77)

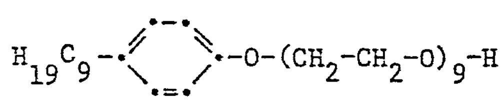

genannt.

Als nicht-ionisches Tensid für die Komponente ($B_2$) des erfindungsgemässen Gemisches sind ebenfalls handelsübliche Produkte aus Aethylenoxyd und Propylenoxyd bevorzugt, die durch Addition von Aethylenoxyd an Additionsprodukte aus Propylenoxyd und Propylenglykol erhältlich sind und Molekulargewichte von etwa 1000 bis etwa 15000 aufweisen. Solche Addukte stellen Blockcopolymerisate dar, die der wahrscheinlichen Formel

(78) $HO-(CH_2-CH_2-O)_x-(CH_2-\underset{CH_3}{\underset{|}{C}H}-O)_y-(CH_2-CH_2-O)_z-H$

entsprechen, worin x, y und z gleiche oder voneinander verschiedene, ganze Zahlen bedeuten. Die Werte für x, y und z hängen vom Molekulargewicht des Copolymerisates ab und stellen nur Durchschnittswerte dar. Besonders bevorzugt sind Copolymerisate mit durchschnittlichen Molekulargewichten von 2000 bis 8000, worin die Durchschnittswerte von x und z je zwischen 2 und 60 und von y zwischen 20 und 80 schwanken und somit der wahrscheinlichen Formel

(79) $HO-(CH_2-CH_2-O)_{2-60}-(CH_2-\underset{CH_3}{\underset{|}{C}H}-O)_{20-80}-(CH_2-CH_2-O)_{2-60}-H$

entsprechen.

Die nicht-ionischen Tenside der Formeln (72), (74), (75) und (78) und vor allem (77) und (79), sind besonders bevorzugt.

Geeignet sind ferner auch Fettsäureester von 3- bis 6-wertigen Alkoholen mit 3 bis 6 Kohlenstoffatomen oder von Mono- oder Disacchariden (Saccharose). Die Fettsäurereste leiten sich z.B. von gesättigten oder ungesättigten Fettsäuren mit vorzugsweise 12 bis 18 Kohlenstoffatomen (Laurin-, Palmitin-, Stearin-, Oelsäure) ab. Genannt seien insbesondere die Sorbitan-Fettsäureester.

Tenside mit intramolekular je einer positiven und negativen Ladung als Komponente ($B_3$), d.h. Tenside ohne überschüssige negative Ladungen werden vorzugsweise als Gemisch mit den Komponenten ($B_1$) und/oder vor allem ($B_2$) und gegebenenfalls ($B_4$) eingesetzt. Insbesondere werden Gemische aus den Komponenten ($B_2$) und ($B_3$) gegenüber den nicht-ionischen Tensiden ($B_2$) für sich allein der Vorzug gegeben. Bei den Tensiden ($B_3$) handelt es sich vor allem um Betaine oder Sulfobetaine, die sich von Imidazolinderivaten oder offenkettigen, aliphatischen Aminen ableiten.

Die als Komponente ($B_3$) erfindungsgemäss eingesetzten, sich von Imidazolinderivaten ableitenden zwitterionischen Betaine oder Sulfobetaine entsprechen vorzugsweise einer der Formeln

(80) 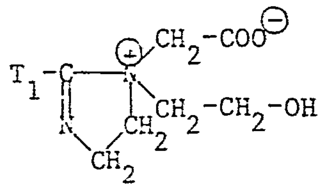

oder

(81) 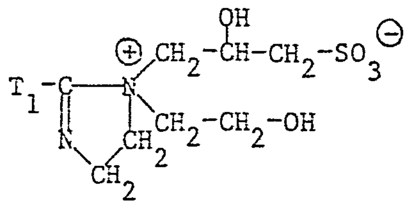 ,

worin $T_1$ die angegebenen Bedeutungen hat.

Als Beispiele spezifischer Vertreter solcher Imidazoliniumbetaine oder -sulfobetaine seien u.a. die Tenside, bzw. technischen Tensidgemische der Formeln

(82) 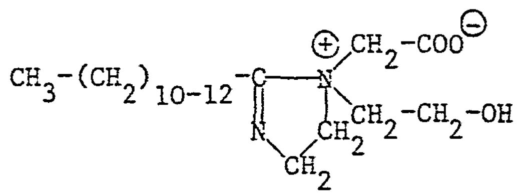

und

(83) 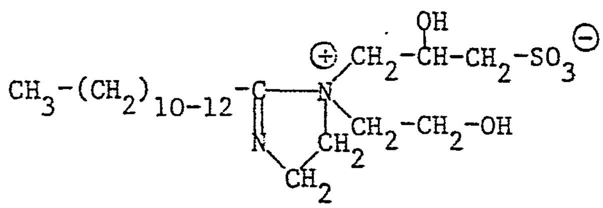

genannt.

Sich von offenkettigen, aliphatischen Aminen ableitende, amphotere Betain- oder Sulfobetainderivate entsprechen bei pH-Werten von höchstens 7,1 ihrer wässrigen Lösungen auch der Formel

(84) 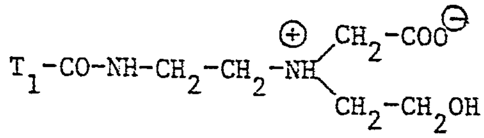 oder

(85) 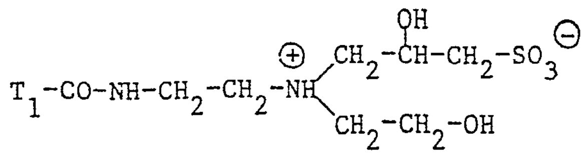 ,

worin $T_1$ die angegebenen Bedeutungen hat,

oder, als spezifische Vertreter, der Formel

(86) 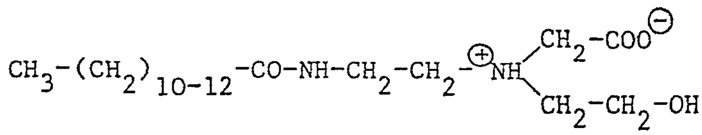 oder

(87) 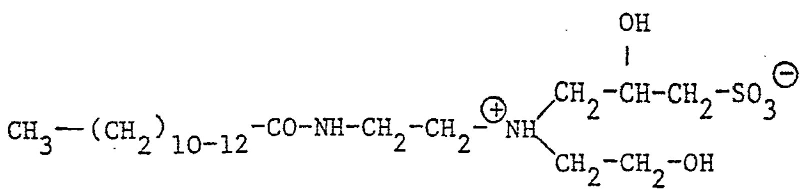 .

Die erfindungsgemäss eingesetzten, sich von einem offenkettigen, aliphatischen Amin ableitenden Betainderivate entsprechenden vorzugsweise einer der Formeln

(88) 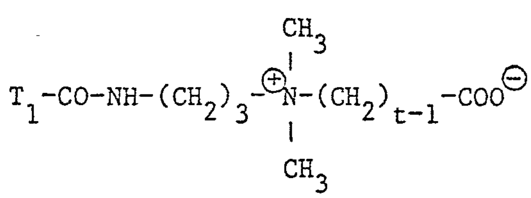 ,

(89) 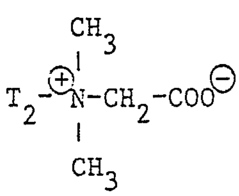 ,

(90) $T_2-\overset{\oplus}{N}H_2-CH_2-(CH_2)_{t-1}-COO^{\ominus}$ ,

(91) $T_2-(NH-CH_2-CH_2)_{t-1}-NH-CH_2-CH_2-\overset{\oplus}{N}H_2-CH_2-COO^{\ominus}$ ,

(92) 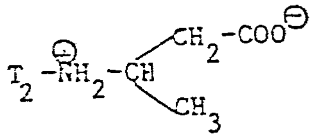 oder

(93) 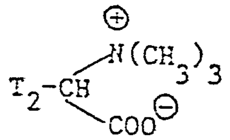 ,

worin $T_1$ und $T_2$ die angegebenen Bedeutungen haben, und t 1 oder 2 bedeutet.

Als Beispiele spezifischer Vertreter solcher Betainderivate seien u.a. die Tenside bzw. technischen Tensidgemische der Formeln

(94) 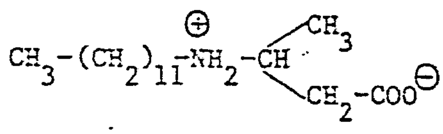 ,

(95) 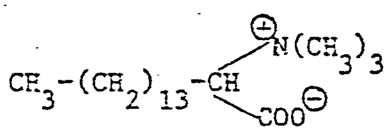 ,

und insbesondere

(96) $CH_3-(CH_2)_{11-13}-\overset{\ominus}{N}H_2-(CH_2)_2-COO^{\ominus}$ ,

(97) 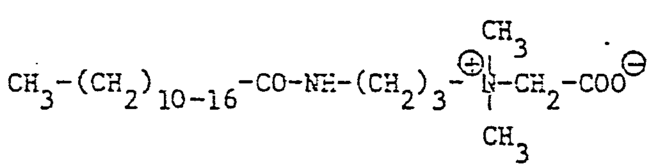 ,

(98) $CH_3-(CH_2)_{10-12}-CH_2-\overset{\oplus}{N}H_2-(CH_2)_2COO^{\ominus}$

und

(99) 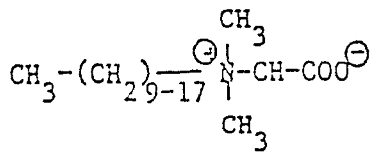

genannt.

Die erfindungsgemäss eingesetzten Sulfobetainderivate, die sich von einem aliphatischen, offenkettigen Amin ableiten, entsprechen vorzugsweise einer der Formeln

(100) 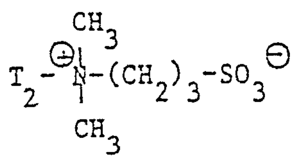 ,

(101) 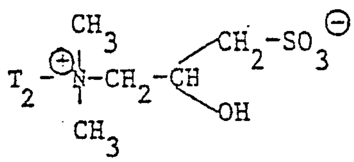 ,

(102) 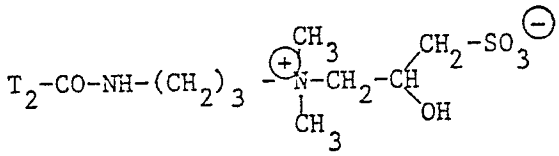

(103) 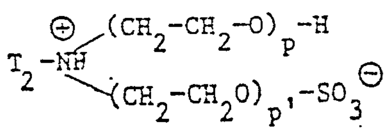 oder

(104) 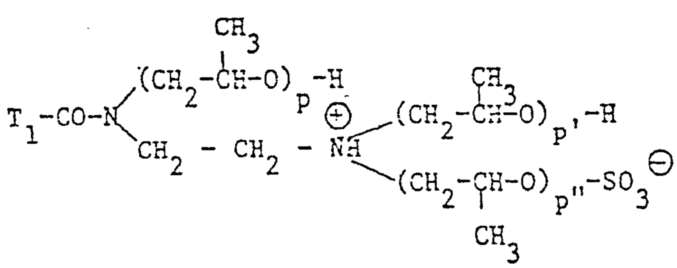 ,

worin $T_1$ und $T_2$ die angegebenen Bedeutungen und p' und p" die für p angegebenen Bedeutungen haben, wobei p, p' und p" voneinander verschieden oder vorzugsweise gleich sind.

Als Beispiele spezifischer Vertreter solcher Sulfobetainderivate seien u.a. die Tenside der Formeln

(105) 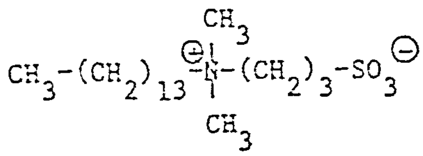 ,

(106) 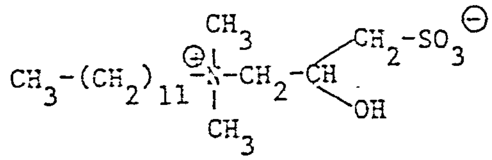 und

(107) 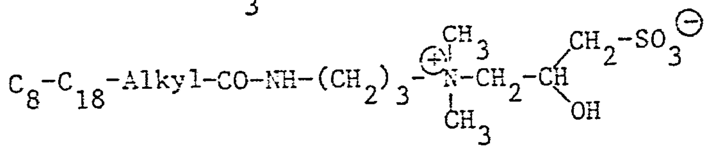

($C_8$-$C_{18}$ = technisches Gemisch aus Kokosfett aus
ca. 15% $C_8$- und $C_{10}$-Alkyl
ca. 40% $C_{12}$-Alkyl
ca. 30% $C_{14}$-Alkyl und
ca. 15% $C_{16}$- und $C_{18}$-Alkyl)
genannt.

Zwitterionische Tenside als Komponente ($B_4$) der erfindungsgemässen Zusammensetzung, die im Gegensatz zur Komponente ($B_3$) überschüssige negative Ladungen enthalten, zeichnen sich dadurch aus, dass sie intramolekular in der Regel eine positive und zwei negative Ladungen aufweisen. Solche Tenside ($B_4$) entsprechen im allgemeinen der Formel

(108) $$X_2^{\ominus} Z^{\oplus}$$ ,

worin $X_2^{\ominus}$ für den Rest von oberflächenaktiven N-Alkyl-α-iminodipropionaten und insbesondere von in 2-Stellung alkylsubstituierten Imidazolinium-dicarbonsäurederivaten stehen und $Z^{\oplus}$ die bei der Formel (19) vorstehend angehende Bedeutungen hat.

Oberflächenaktive, alkylsubstituierte Iminodipropionate entsprechen vorzugsweise der Formel

(109) 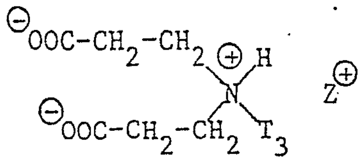 ,

worin $T_3$ und $Z^{\oplus}$ die angegebenen Bedeutungen haben.

Als spezifisches Beispiel seien die Tenside der Formeln

(110) 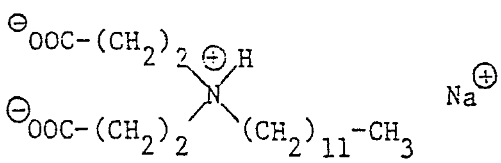

und

(111) 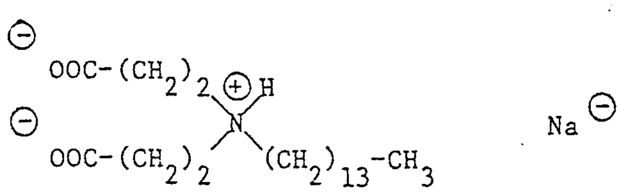

oder technische Gemische aus den Tensiden der Formeln (110) und (111) genannt.

Bei den bevorzugten, zwitterionischen Imidazolinium-Tensiden der angegebenen Art handelt es sich vor allem um solche der Formel

(112) 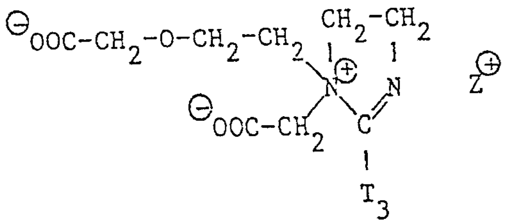 ,

worin $T_3$ und $Z^{\oplus}$ die angegebenen Bedeutungen haben. Als spezifische Beispiele seien die Tenside der Formel

(113) 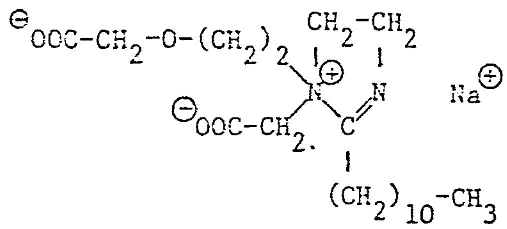 oder

(114) 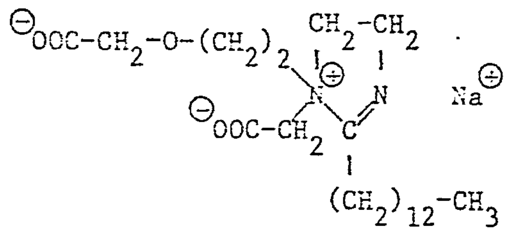

oder deren technische Gemische erwähnt.

Ferner entsprechen auch in einer anderen bevorzugten Ausführungsart die amphoteren anionischen Tensiden der Formel (108) bei pH-Werten von höchstens 7,1 ihrer wässrigen Lösungen der Formel

(115) 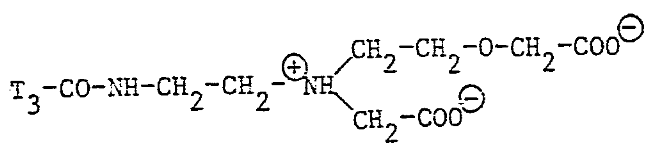 ,

worin $T_3$ die angegebenen Bedeutungen hat, oder, als spezifische Vertreter, der Formel

(116) 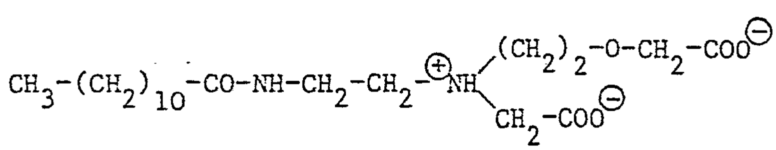 oder

(117) 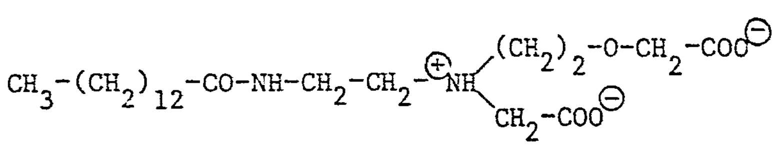

Im Vordergrund des Interesses stehen als Tenside der erfindungsgemässen Zusammensetzungen Addukte aus Aethylenoxyd und Propylenoxyd eines Molekulargewichtes von 2000 bis 8000, die der Formel (79) entsprechen, vor allem oberflächenaktive, anionische Sulfate der Formel (19), Sulfonate der Formel (29) und Sulfobernsteinsäurederivate der Formel (42) für sich allein oder in Gemisch mit oberflächenaktiven, anionischen, zwitterionischen Iminodipropionaten der Formel (109) oder Imidazoliniumdicarbonsäurederivaten der Formel (112) und insbesondere oberflächenaktive, anionische Sulfate, Sulfonate oder Sulfobernsteinsäurederivate der Formel (26), (27), (36) oder (49).

Bei den Fettsäuren mit 12 bis 22 Kohlenstoffatomen als Komponente (C) der erfindungsgemässen Zusammensetzung handelt es sich im allgemeinen um die bei der Definition des Restes $T_1COO$- in Formel (16) vorstehend erwähnten Fettsäuren, bzw. Fettsäure-Gemische. Diese Fettsäuren oder deren Gemische können je nach pH-Wert der Zusammensetzungen teilweise als Salze vorliegen. Bei dem pH-Wert von höchstens 7,1 des erfindungsgemässen Zusammensetzungen liegen jedoch jedenfalls die Festtsäuren als Komponente (C) in den erfindungsgemässen Zusammensetzungen mindestens teilweise in Form der freien Säuren vor. Bevorzugt weisen die Fettsäuren 16 bis 18 Kohlenstoffatomen auf, wobei die Oel-, Stearin- und Palmitinsäure ihrer besonderen Zugänglichkeit wegen im Vordergrund des Interesses stehen.

In bevorzugter Kombination enthalten die erfindungsgemässen Zusammensetzungen, die im Vordergrund des Interesses stehen,

(A) ein Poly(dimethylbutenylammoniumchlorid)-$\alpha,\omega$-bis-(triäthanolammoniumchlorid) eines Molekulargewichtes von 1000 bis 5000 der Formel (3),

ein Copolymerisat aus Dimethyldiallylammoniumchlorid und Acrylamid eines Molekulargewichtes von $10^4$ bis $3 \cdot 10^6$ mit durchschnittlich 20 bis 90 Mol-% wiederkehrenden Strukturelementen der Formel (4), worin n 2 ist, und durchschnittlich 10 bis 80 Mol-% wiederkehrenden Strukturelementen der Formel (5),

ein Copolymerisat aus Acrylamid und quaternären Ammoniumsalzen der Acrylsäurereihe einer Molekulargewichtsverteilung von $10^4$ bis $10^9$, wobei das Molekulargewicht von etwa 10 bis 60 Gewichtsprozent des Copolymerisats $10^7$ bis $10^9$ beträgt, mit durchschnittlich 1 bis 90 Mol % wiederkehrenden Strukturelementen der Formel (6) und durchschnittlich 10 bis 99 Mol % wiederkehrenden Strukturelementen der Formel (5)

oder ein Copolymerisat aus aliphatischen Dicarbonsäuren und Polyalkylenpolyaminen eines Molekulargewichtes von $10^3$ bis $10^4$ mit wiederkehrenden Strukturelementen der Formel (13),

($B_1$) ein Addukt aus Aethylenoxyd und Propylenoxyd eines Molekulargewichtes von 2000 bis 8000 der Formel (79) oder ($B_2$) einen anionischen Sulfat, Sulfonat oder Sulfobersteinsäurederivat einer der Formeln (19), (29 oder (42) allein oder in Gemisch mit einem anionischen, zwitterionischen, alkylsubstituierten Iminodipropionat oder Imidazoliniumdicarbonsäurederivat einer der Formeln (109) oder (112) und

(C) eine Fettsäure mit 16 bis 14 Kohlenstoffatomen

oder insbesondere

(A) ein Copolymer aus Acryl- oder Methacrylamid und quaternären Ammoniumsalzen der Acrylsäurereihe einer Molekulargewichtsverteilung von $10^4$ bis $10^9$, wobei das Molekulargewicht von 12 bis 50 Gewichtsprozent des Copolymers $10^7$ bis $10^9$ beträgt, mit durchschnittlich 20

bis 35 Mol % wiederkehrenden Strukturelementen der Formel (10) und durchschnittlich 65 bis 80 Mol % wiederkehrenden Strukturelementen der Formel (5),

($B_1$) ein anionisches Tensid einer der Formeln (26), (27), (36) oder (49) und

(C) Oel-, Stearin- oder Palmitinsäure.

Bevorzugt beträgt der pH-Wert der erfindungsgemässen Zusammensetzungen 3,50 bis 6,95 und insbesondere 5,0 bis 6,8.

Bevorzugte erfindungsgemässe Zusammensetzungen enthalten die Komponenten (A), ($B_1$) bis ($B_4$) und (C) in einem Gewichtsverhältnis (A):($B_1$) bis ($B_4$):(C) von 1:(4 bis 250):(0,005 bis 40), insbesondere 1:(10 bis 90):(0,1 bis 5).

Zur Herstellung der erfindungsgemässen Zusammensetzung wird z.B. so verfahren, dass man ein in wässerigen Tensidsysteme lösliches oder mikroemulgierbares, polymeres, quaternäres Ammoniumsalz, das eine Molekulargewichtsverteilung von $10^3$ bis $10^9$ aufweist, als Komponente (A) mit mindestens einem anionischen Tensid ($B_1$), einem nicht-ionischen Tensid ($B_2$), einem Tensid ($B_3$) mit intramolekular je einer positiven und negativen Ladung oder einem Tensid ($B_4$) mit intramolekular einer positiven und zwei negativen Ladungen, wobei die Tenside ($B_3$) und ($B_4$) als Gemisch mit mindestens einem der Tenside ($B_1$) und ($B_2$) eingesetzt werden, und mindestens einer Fettsäure mit 12 bis 22 Kohlenstoffatomen als Komponente (C) bei 10 bis 90° C und einem pH-Wert von höchstens 7,1 vermischt, wobei man gleichzeitig, sofern ein anionisches Tensid ($B_1$) für sich allein oder in Gemisch mit einem Tensid ($B_4$) mit intramolekular einer positiven und zwei negativen Ladungen eingesetzt wird, die Tenside ($B_1$) und gegebenenfalls ($B_4$) mit

dem polymeren, quaternären Ammoniumsalz als Komponente (A) unter Ionenaustausch mindestens teilweise umsetzt.

Somit werden bei der Herstellung der Zusammensetzung die polymeren, quaternären Ammoniumsalze als Komponente (A), die als Homo- oder vorzugsweise als Copolymerisate vorliegen, nach an sich bekannten Methoden mit mindestens einem anionischen Tensid als Komponente ($B_1$) einer der Formeln (15) bis (71), einem nicht-ionischen Tensid als Komponente ($B_2$) einer der Formeln (72) bis (79), einem Tensid mit intramolekular je einer positiven und negativen Ladung als Komponente ($B_3$) einer der Formeln (8) bis (107) und/oder einem Tensid mit intramolekular einer positiven und zwei negativen Ladungen als Komponente ($B_4$) einer der Formeln (109) bis (117), wobei die Komponenten ($B_3$) und ($B_4$) als Gemisch mit einem der Komponente ($B_1$) und ($B_2$) eingesetzt werden, und mit einer Fettsäure mit 12 bis 22 Kohlenstoffatomen als Komponente (C), vorzugsweise bei Raumtemperatur (15 bis 25° C) miteinander vermischt, oder, sofern ein anionisches Tensid für sich allein oder als Gemisch mit einem Tensid mit intramolekular einer positiven und zwei negativen Ladungen als Komponenten ($B_1$) und gegebenenfalls ($B_4$) eingesetzt werden, bei bevorzugten, erhöhten Temperaturen von 15 bis 90° C, vorzugsweise 20 bis 40°C, während 10 bis 120, vorzugsweise 30 bis 100, insbesondere 60 bis 90 Minuten, mindestens teilweise umgesetzt, wobei in der Regel ein Ueberschuss von vorzugsweise etwa 1 bis 500, vor allem 10 bis 120, insbesondere 20 bis 100 Mol an anionisches Tensid und gegebenenfalls an Tensid mit intramolekular einer positiven und zwei negativen Ladungen einer der Formeln (15) bis (71) und gegebenenfalls (109) bis (117) bezogen auf 1 Mol der quaternären Strukturelemente der Formel (2), (3), (4), (6), (10), (12), (13) oder (14) der homo- oder vorzugsweise copolymeren Ammoniumsalze eingesetzt wird und die Umsetzung unter Ionenaustausch des Anions der genannten Strukturelemente mit dem Anion $X_1^{\ominus}$ der Formel (15) und gegebenenfalls $X_2^{\ominus}$ der Formel (108) des anionischen Tensides und gegebenenfalls des Tensides mit einer positiven und zwei negativen Ladungen mindestens teilweise erfolgt.

In einer bevorzugten Ausführungsart des erfindungsgemässen Herstellungsverfahrens der Zusammensetzungen wird bei Verwendung eines anionischen Tensids allein oder in Gemisch mit einem Tensid mit intramolekular einer positiven und zwei negativen Ladungen als Komponente ($B_1$) und gegebenenfalls ($B_4$) zuerst ein Teil, vorzugsweise ca. 1/2 bis 3/4 der als wässrige Lösung vorliegenden Tenside zur Gesamtmenge der als wässrige Lösung vorliegenden Komponente (A) innerhalb 30 bis 100, insbesondere 60 bis 90 Minuten bei 15 bis 90, insbesondere 20 bis 40° C gegeben, wobei die vollständige oder partielle Umsetzung der Komponenten (A) und ($B_1$) oder (A) und ($B_1$) und ($B_4$) unter Ionenaustausch durchgeführt wird, worauf der verbleibende Teil, vorzugsweise ca. 1/4 bis 1/2 der wässrigen Tensidlösung mit der Komponente (C) zu einer feinteiligen, wässrigen Suspension vermischt wird, die zuletzt in die wässerige Lösung gegeben wird, welche die mit dem Tensid ($B_1$) und gegebenenfalls ($B_4$) mindestens teilweise umgesetzte Komponente (A) und das überschüssige, nicht umgesetzte Tensid enthält.

Bei ihrer Verwendung in der Kosmetikindustrie werden die erfindungsgemässen Gemische der Komponenten (A), ($B_1$) bis ($B_4$) und (C) vorzugsweise als haarkosmetisches Mittel, insbesondere Haarshampoos eingesetzt.

Die erfindungsgemässen kosmetischen Mittel, vorzugsweise haarkosmetische Mittel, die insbesondere als Haarshampoos einsetzbar sind, liegen in ihrer bevorzugten Ausführungsform als wässrige Lösungen vor, die z.B.

0,1 bis 2,0, vorzugsweise 0,2 bis 1,0 Gewichtsteile, berechnet als Wirksubstanz, der Polymerisate der angegebenen Art als Komponente (A), 8 bis 25, vorzugsweise 10 bis 18 Gewichtsteile, berechnet als Wirksubstanz, der Tenside der angegebenen Art als Komponenten ($B_1$) bis ($B_4$),

0,01 bis 4,0 vorzugsweise 0,1 bis 1,0 Gewichtsteile, berechnet als Wirksubstanz, einer Fettsäure der angegebenen Art als Komponente (C) und

gegebenenfalls kosmetische Hilfsmittel als fakultative Komponente (D) enthalten,

wobei diese Mittel aus den Komponenten (A) ($B_1$) bis ($B_4$), (C) und gegebenenfalls (D) mit entionisiertem Wasser auf insgesamt 100 Gewichtsteile verdünnt sind.

Die fakultativen, kosmetischen Hilfsmittel als Komponente (D) sind handelsübliche Mittel, wie sie in der Haarkosmetik eingesetzt werden. In Betracht kommen z.B. Tenside, die von den als Komponenten ($B_1$) bis ($B_4$) eingesetzten Tensiden der angegebenen Art verschieden sind, wie beispielsweise Polyglycerolester und Polyglykolester von Fettsäuren, insbesondere Polyglycerinoleate, im weiteren auch Polyglykole und Schaumstabilisatoren (z.B. Fettsäurepolyalkanolamide), Verdickungsmittel natürlicher oder synthetischer Herkunft (z.B. Hydroxypropylmethylcellulose und Polyacrylsäure), Opalisierungsmittel (z.B. Fettsäuremonoalkanol-amide oder vorzugsweise Glycerinmonostearat), Hautschutzmittel (z.B. Eiweisshydrolysate und Allantoine), ferner auch u.a. Konservierungsmittel, Parfüms und Perlglanzmittel.

Erforderlichenfalls werden die kosmetischen Mittel auf den pH-Wert von höchstens 7,1 eingestellt, was zweckmässig durch einen Zusatz von wässrigen Lösungen von z.B. Natriumhydroxyd oder Zitronensäure erreicht werden kann. In der Regel ist der Zusatz einer wässrigen Zitronensäurelösung notwendig, um den bevorzugten pH-Wert von 3,50 bis 6,95, insbesondere 5,0 bis 6,8 der Zusammensetzungen in Form von wässrigen haarkosmetischen Mitteln einzustellen.

Bei der Applikation der haarkosmetischen Mittel, vorzugsweise auf keratinhaltigen Materialien, insbesondere menschlichem Haar, wird im Haarbehandlungsverfahren das vorstehend beschriebene, wässrige, haarkosmetische Mittel in Form eines Shampoos auf mit Leitungswasser angefeuchtetem Haar, in der Regel bei Raumtemperatur bis leicht erhöhter Temperatur, z.B. 20 bis 40°C, aufgebracht und das Haar anschliessend shampooniert und gleichzeitig konditioniert. Das so zu behandelnde Haar kann ebenfalls in Form von Perücken oder Toupets vorliegen.

Der wesentliche Vorteil der vorliegenden Erfindung liegt darin, dass bei der Applikation der haarkosmetischen Mittel, welche neben polymeren, quaternären Ammoniumsalzen der angegebenen Art als Komponente (A) und Tensiden der angegebenen Art als Komponenten ($B_1$) bis ($B_4$) in neuartiger Kombination auch leicht zugängliche, preisgünstige und umweltfreundliche Fettsäuren als Komponente (C) enthalten, nicht nur eine gute Reinigung, sondern auch gleichzeitig ausgezeichnete Konditioniereffekte auf dem behandelten Haar erzielt werden. So weisen die mit der erfindungsgemässen Zusammensetzung behandelten Haare antistatische Eigenschaften und eine sehr gute Kämmbarkeit (nass und trocken) auf. Zudem ist bei wiederholter Verwendung der erfindungsgemässen Zusammensetzung keinerlei unerwünschter Akkumuliereffekt unter Beeinträchtigung des Griffs, der Fülle und des Glanzes der behandelten Haare zu beobachten. Im Vergleich zu den Konditioniereffekten auf Haaren, die mit herkömmlichen kosmetischen Mitteln behandelt werden, weisen die mit den erfindungsgemässen haarkosmetischen Mitteln behandelten Haare somit in unerwarteter Weise ausserordentlich gute Konditioniereffekte auf. Die üblichen kosmetischen Mittel enthalten zwar auch Mischungen

aus Tensiden und quaternären Ammoniumsalzen, jedoch nicht deren neuartige und erfinderische Kombination mit Fettsäuren gemäss vorliegender Erfindung. Als weiteren Vorteil sei erwähnt, dass beim Shampoonieren der Haare die erfindungsgemässen Zusammensetzungen einen Schaum der erwünschten, cremigen Beschaffenheit entwickeln, der stabiler, d.h. länger bestehen bleibt als beim Shampoonieren der Haare mit bekannten Zusammensetzungen. Als zusätzlichen Vorteil sei noch erwähnt, dass die Haarreinigung einstufig ohne Mitverwendung eines Nachspülmittels in einer aufwendigen zweiten Stufe erfolgt.

In den nachfolgenden Herstellungsvorschriften und Beispielen angegebene Teile und Prozente sind Gewichtseinheiten.

Herstellungsvorschriften für die Komponente (A)

Vorschrift A: (Wasser-in-Oel Emulsionscopolymerisat). Die folgenden drei Lösungen werden in sauerstofffreier, inerter Stickstoffatmosphäre vorbereitet:

Lösung I (Oelphase)

In einem Doppelmantelreaktionsgefäss werden

500 Teile eines verzweigten Paraffinöls (technisches Gemisch, Molekulargewicht 171, Siedebereich 188 - 206°C) vorgelegt.

140 Teile einer 2,5 %igen Lösung eines synthetischen Kautschuks aus Polyisopren (Emulsionsstabilisator) in Paraffinöl der angegebenen Art und anschliessend

78 Teile Sorbitanmonooleat (Wasser-in-Oel Emulgator) werden zum vorgelegten Paraffinöl unter Rühren bei 20°C gegeben.

Man erhält eine klare, gelbe Lösung.

Lösung II (wässrige Phase)

568,6 Teile Acrylamid (8 Mol) werden bei 20°C in
700 Teilen entionisiertem, sauerstofffreiem Wasser gelöst. Zu dieser Lösung werden
220 Teile Natriumchlorid unter Rühren eingetragen und anschliessend
1133,2 Teile einer 50%igen, wässrigen Lösung aus Methacryloyl-oxy-äthyl-trimethylammonium-methylsulfat (2 Mol) gegeben.

Man erhält eine klare farblose Lösung.

Lösung III (Initiatorlösung)

0,66 Teile Natriumsulfit werden in
40 Teilen entionisiertem, sauerstofffreiem Wasser gelöst.

Copolymerisationsreaktion

Unter intensivem Rühren (3000 U/min) wird innerhalb von 10 Minuten in inerter Stickstoffatmosphäre die Lösung II zur vorgelegten Lösung I bei 20° C gegeben. Man erhält eine homogene, weisse Emulsion, die bei 20° C so lange unter Rühren gehalten wird, bis die Viskosität einer Emulsionsprobe 14000 mPa.s (Brookfield Viskosimeter LV, Spindel 3, 6 U/min, 25° C) beträgt, was im allgemeinen 10 Minuten in Anspruch nimmt. Hierauf wird das Reaktionsgemisch unter Rühren bei 300 U/min innerhalb von 30 Minuten auf 40° C aufgeheizt. Mittels einer Dosierpumpe wird nun die Lösung III innerhalb von 150 Minuten zum Reaktionsgemisch gegeben, wobei die Temperatur durch Kühlen auf 40 bis 41° C gehalten wird. Nach Beendigung der Initiatorlösung-Zugabe wird das Reaktionsgemisch bei 40° C und 300 U/min so lange unter Rühren gehalten, bis die Viskosität einer Emulsionsprobe auf 7600 mPa.s.

(Brookfield Viskosimeter LV, Spindel 1, 60 U/min, 25°C) absinkt, was im allgemeinen eine Stunde in Anspruch nimmt.

Aufarbeitung

Die erhaltene Emulsion des Copolymerisates wird unter Rühren zu 24000 Teilen Aceton bei 20°C gegeben und das Copolymerisat ausgefällt. Das ausgefällte Copolymerisat wird abfiltriert und während 2 Tagen unter vermindertem Druck bei 40°C getrocknet. Man erhält 1100 Teile eines Copolymerisates, das in beliebiger Reihenfolge 80 Mol% Strukturelemente der Formel (5) und
20 Mol% Strukturelemente der Formel

(118) 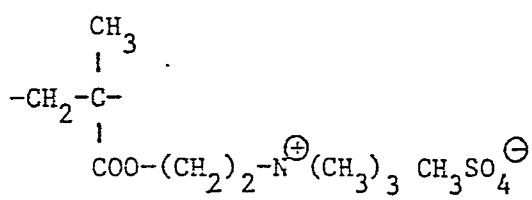

enthält, wobei das Molekulargewicht von 37% des Copolymerisates zwischen $10^7$ und $10^9$ liegt.

Die molekulare Gewichtsverteilung wird mittels Gelpermeationschromatographie bestimmt, wobei als Trägermaterial FRACTOGEL® Typ OR-PVA eingesetzt wird, welches zweckmässig in Formamid als Lösungsmittel gequollen wird. Einzelheiten über das Trägermaterial sind aus dem Lehrbuch "Modern Size-Exclusion Chromatography" von W.W. Yau, J.J. Kirkland and D.D. Bly, Seiten 166 bis 173, Verlag John Wiley & Sons (1979) zu entnehmen. Als Detektor dient ein Differenzialrefraktometer.

Die Auswertung der GPC-Chromatogramme erfolgt anhand einer Eichkurve, die mit Polystyrol, Polymethylmethacrylat und den Standard-Polymeren SEPHADEX DEXTRAN® erstellt wird. Die Konzentrationen werden über die unkorrigierten Fächenprozente ermittelt, wobei die Summe aller eluierten Komponenten 100 % beträgt.

Vorschrift B: (Lösungscopolymerisat)

Die folgenden zwei Lösungen werden in sauerstofffreier inerter Stickstoffatmosphäre vorbereitet:

Lösung I (Monomerlösung)

In einem Doppelmantelgefäss werden

71,1 Teile Acrylamid (1 Mol) und

141,7 Teile einer 50%igen, wässrigen Lösung aus Methacryloyl-oxyäthyltrimethylammonium-methylsulfat (0,25 Mol) bei 20°C in

228 Teilen entionisiertem, sauerstofffreiem Wasser gelöst.
Man erhält eine klare farblose Lösung.

Lösung II (Initiatorlösung)

0,2 Teile Ammoniumperoxodisulfat werden in 150 Teilen deionisiertem sauerstofffreiem Wasser gelöst.

Copolymerisationsreaktion

Unter Rühren wird innerhalb von 1 Minute in inerter Stickstoffatmosphäre die Hälfte der Lösung II zur vorgelegten Lösung I bei 35° C gegeben. Nach 6 Stunden wird die Reaktionslösung auf 50° C erwärmt und die zweite Hälfte von Lösung II gegeben. Das Reaktionsgemisch wird solange unter Rühren gehalten, bis nach 2 bis 3 Stunden eine hochviskose Lösung entstanden ist. Ohne Rühren wird das Reaktionsgemisch stehen gelassen. Nach 24 Stunden lässt man das entstandene farblose Gel abkühlen.

Aufarbeitung

Das Gel wird zerkleinert und in 1350 Teilen deionisiertem Wasser gelöst. Die hochviskose Lösung wird dann zu 18000 Teilen Aceton in dünnem Strang bei 20°C eingepresst und das Copolymerisat ausgefällt.

Das Copolymerisat wird dann abfiltriert und nochmals in 1800 Teilen Aceton geknetet, bis es hart und spröde wird. Das Copolymerisat wird wieder abfiltriert und während 2 Tagen unter vermindertem Druck bei 40° C getrocknet. Man erhält 110 Teile eines Copolymerisats, das in beliebiger Reihenfolge 80 Mol% Strukturelemente der Formel (5) und 20 Mol% Strukturelemente der Formel (118) enthält, wobei das Molekulargewicht von 12 % des Copolymerisats zwischen $10^7$ und $10^9$ liegt. Hierbei wird diese Molekulargewichtsverteilung wie in Vorschrift A angegeben bestimmt.

<u>Vorschrift C:</u> (Behandlung eines Handelsproduktes)

100 Teile eines im Handel erhältlichen Copolymerisates, das zu 75 Mol-% aus Strukturelementen der Formel (5) und zu 25 Mol-% aus Elementen der Formel

(119)
$$\begin{array}{l} \qquad\quad CH_3 \\ \qquad\quad\ | \\ -CH_2-C- \qquad\qquad \oplus \qquad\qquad Cl^{\ominus} \\ \qquad\quad\ | \\ \qquad\quad COO-(CH_2)_2-N-(CH_3)_3 \end{array}$$

besteht, wobei das Molekulargewicht von 20% des Copolymerisates zwischen $10^7$ und $10^9$ liegt, werden pulverisiert und in einem Gemisch von 400 Teilen Methanol und 100 Teilen Wasser bei Raumtemperatur während 30 Minuten unter Rühren gehalten. Weitere 500 Teile Methanol werden zugesetzt und das Gemisch während noch 5 Minuten unter Rühren gehalten und dann eine Stunde stehen gelassen. Der gelige Brei wird dann in einer Drucknutsche unter einem Druck von 3 bar filtriert. Die gelige Masse wird anschliessend in 100 Teilen Methanol aufgeschlämmt und wieder unter Druck abfiltriert. Nach dreimaliger Behandlung mit Methanol wird das Produkt während 24 Stunden unter vermindertem Druck bei 40° C getrocknet. Das Molekulargewicht von 45 % des erhaltenen, umgefällten Copolymerisats liegt zwischen $10^7$ und $10^9$, wobei die Molekulargewichtsverteilung des behandelten Copolymerisats und des Ausgangspolymers (Handelsprodukt) wie in Vorschrift A angegeben bestimmt wird.

Beispiel 1:

Lösung A: 2 Teile des quaternären, polymeren Ammoniumsalzes gemäss Vorschrift C (0,0024 Mol Strukturelemente der Formel (119)) werden bei 20°C in kleinen Portionen innerhalb von 30 Minuten in 100 Teilen entionisiertem Wasser eingetragen. Es entsteht eine viskose Lösung.

Lösung B: 100 Teile des anionischen Tensides der Formel (27) (0,24 Mol oder 100 Mol pro Mol quaternäre Strukturelemente der Formel (119) des Ammoniumsalzes gemäss Vorschrift C) werden mit 400 Teilen entionisiertem Wasser bei 25°C zu 500 Teilen Tensidlösung verdünnt.

Lösung C: Die Lösung A des quaternären, polymeren Ammoniumsalzes (102 Teile) wird nun zu 350 Teilen der Tensidlösung B innerhalb von 80 Minuten bei 35° C gegeben, wobei gleichzeitig die Umsetzung der quaternären Strukturelemente der Formel (119) des polymeren Ammoniumsalzes mit dem eingesetzten, anionischen Tensids unter Ionenaustausch mindestens teilweise erfolgt. Man erhält 452 Teile einer wässrigen, schwach opalisierenden und viskosen Lösung.

Suspension D: In die verbleibende Portion (150 Teile) der Tensidlösung B werden 5 Teile Stearinsäure bei 80°C innerhalb von 30 Minuten unter Rühren eingetragen. Man erhält 155 Teile einer feinteiligen Suspension, die auf 20°C abgekühlt wird.

Erfindungsgemässe Zusammensetzung: Die Suspension D wird nun unter Rühren bei 20°C innerhalb von 15 Minuten in die Lösung C eingetragen. Man erhält 607 Teile einer wässrigen Emulsion, die durch Zusatz einer 10 %igen, wässrigen Zitronensäurelösung auf den pH-Wert von 5,0 eingestellt und mit entionisiertem Wasser auf 1000 Teile verdünnt wird.

Applikation der Zusammensetzung: Nun wird die verdünnte Emulsion auf eine mit Leitungswasser angefeuchtete Perücke aus ungebleichtem und ungefärbtem, braunem Menschenhaar europäischer Herkunft bei 40°C in dreimaliger Applikation à 2 Shampoonierungen im sogenannten Halbseitentest aufgebracht, worauf das Perückenhaar bei dieser Temperatur shampooniert und gleichzeitig konditioniert wird. Nach jeder Shampoonierung wird das Haar mit Leitungswasser von 20 bis 40°C kurz nachgespült. Im Halbseitentest wird nur die eine Hälfte der Perücke mit der vorstehend genannten Lösung shampooniert und konditioniert, während die andere Hälfte der Perücke mit einer Lösung unter gleichen Bedingungen shampooniert wird, die kein erfindungsgemässes Gemisch aus dem Ammoniumsalz, der Fettsäure und dem Tensid, sondern nur das Tensid enthält. In dieser sogenannten Leerformulierung wird die Menge an Ammoniumsalz und an Fettsäure durch die entsprechende Menge Tensid ersetzt, so dass z.B. die Leerformulierung als Vergleich 10,7 % des Tensids der Formel (27) enthält, wobei der pH-Wert der Leerformulierung ebenfalls mit der wässrigen 10 %igen Zitronensäurelösung auf den pH-Wert von 5,0 eingestellt wird. Nach jeder Applikation wird die Nass- und Trockenkämmbarkeit der erfindungsgemäss behandelten Perückenhälfte im Vergleich zu der mit der Leerformulierung behandelten Perückenhälfte mit Hilfe der nachfolgenden Notenskala beurteilt:

+3 viel besser als die Leerformulierung
+2 besser als die Leerformulierung
+1 etwas besser als die Leerformulierung
0 kein Unterschied zur Leerformulierung
-1 etwas schlechter als die Leerformulierung
-2 schlechter als die Leerformulierung
-3 viel schlechter als die Leerformulierung.

Mit Hilfe der gleichen Notenskala werden die Griffeigenschaften der behandelten Perückenhälfte in trockenem und nassem Zustand in Vergleich zu der mit der Leerformulierung behandelten Perückenhälfte beurteilt. Je höher die Griffnoten liegen, desto geschmeidiger fällt der Griff aus. Die gleiche Notenskala wird auch benützt, um die Schaumeigenschaften der erfindungsgemässen Shampoozusammensetzung in Vergleich zu den Schaumeigenschaften der Leerformulierung zu beurteilen. Je höher die Schaumeigenschaftsnoten liegen, desto cremiger und stabiler ist der Schaum beim Shampoonieren der Haare.

Die erzielten Kämmbarkeits-, Griff- und Schaumeigenschaftsnoten sind in der nachfolgenden Tabelle I zusammengefasst.

Tabelle I

| | nach der 1. Applikation | nach der 2. Applikation | nach der 3. Applikation |
|---|---|---|---|
| Nasskämmbarkeit | +(2-3) | +3 | +3 |
| Trockenkämmbarkeit | +(1-2) | +2 | +2 |
| Griff (nass) | +(0-1) | +(1-2) | +(1-2) |
| Griff (trocken) | +1 | +(1-2) | +(1-2) |
| Schaumeigenschaften | +(2-3) | +(2-3) | +(2-3) |

Zudem weist die erfindungsgemäss behandelte Perückenhälfte bessere antistatische Eigenschaften auf als die mit der Leerformulierung behandelte Perückenhälfte. Die antistatischen Effekte werden qualitativ erfasst, indem die Neigung der Haare zum "Wegfliegen" (fly away) subjektiv beurteilt wird. Auf beiden Perückenhälften können zudem keinerlei Akkumuliereffekte nach 3 Applikationen beobachtet werden

Aehnliche Ergebnisse werden erzielt, wenn man an Stelle von 2 Teilen des quaternären, polymeren Ammoniumsalzes gemäss Vorschrift C jeweils 2 Teile des quaternären, polymeren Ammoniumsalzes gemäss Vorschrift A oder B oder jeweils 5 Teile der folgenden, im Handel erhältlichen quaternären Ammoniumsalze einsetzt:

- Quaternäres Cellulosderivat, dessen Celluloseeinheiten mit einem 1-Trimethylammonium-2-hydroxypropyl-Rest veräthert sind (POLYMER JR 400®)
- Copolymerisat, das Strukturelemente der Formel (4), worin n 2 ist, und der Formel (5) und welches ein Molekulargewicht von etwa $2 \cdot 10^6$ aufweist (MERQUAT 550®)
- Copolymerisat, das höchstens 5 Mol-% Strukturelemente der Formel (10) und mindestens 95 Mol-% Strukturelemente der Formel (5), und welches ein Molekulargewicht von $10^4$ bis $10^6$ aufweist (RETEN 210®)
- Copolymerisat, das Strukturelemente der Formel (13) aufweist (CARTARETIN F-4®)
- Copolymerisat, das Strukturelemente der Formel (3) aufweist (ONAMER M®)

Ebenfalls werden ähnliche Ergebnisse erzielt, wenn man an Stelle von 100 Teilen des anionischen Tensides der Formel (27) 100 Teile des nicht-ionischen Tensids der Formel (79) oder eines anionischen Tensids einer der Formeln (26), (36) oder (49) einsetzt.

Vergleichsversuch

Zu Vergleichszwecken wird eine analoge Zusammensetzung hergestellt, die jedoch keine Stearinsäure, sondern lediglich 100 Teile des anionischen Tensides der Formel (27) und 2 Teile des quaternären, polymeren Ammoniumsalzes gemäss Vorschrift C, wobei die Umsetzung der quaternären Strukturelemente der Formel (119) des polymeren Ammoniumsalzes mit dem Tensid der Formel (27) wie in Beispiel 1 angegeben ebenfalls mindestens teilweise erfolgt. Die Zusammensetzung wird durch Zusatz einer 10 %igen, wässrigen Zitronensäurelösung ebenfalls auf den pH-Wert von 5,0 eingestellt und mit entionisiertem Wasser auf 1000 Teile verdünnt. Die Applikation der Zusammensetzung erfolgt wie in Beispiel 1 angegeben, wobei die folgenden,

in den nachstehenden Tabelle II angegebenen Kämmbarkeits-, Griff- und Schaumeigenschaftsnoten im Vergleich zur Leerformulierung erzielt werden.

Tabelle II

| | nach der 1. Applikation | nach der 2. Applikation | nach der 3. Applikation |
|---|---|---|---|
| Nasskämmbarkeit | +1 | +1 | +(1-2) |
| Trockenkämmbarkeit | +(0-1) | +(0-1) | +1 |
| Griff (nass) | +(0-1) | +(0-1) | +1 |
| Griff (trocken) | +1 | 0 | +1 |
| Schaumeigenschaften | +(1-2) | +(1-2) | +(1-2) |

Der Vergleich der vorliegenden Tabelle II mit der in Beispiel 1 angegebenen Tabelle I lässt erkennen, dass durch Zusatz der Stearinsäure zu bekannten Zusammensetzungen aus polymeren Ammoniumsalzen gemäss Vorschrift C, die mindestens teilweise mit dem anionischen Tensid der Formel (27) umgesetzt sind und einen Ueberschuss an unumgesetztes Tensid der Formel (27) enthalten, wie sie z.B. in der europäischen Patentanmeldung 45 720 beschrieben sind, eine signifikante Erhöhung der an sich guten Kämmbarkeits-, Griff- und Schaumeigenschaften bewirkt. Auch die qualitativ beurteilen, antistatischen Eigenschaften der mit der erfindungsgemässen Zusammensetzung behandelten Perückenhälfte ist wesentlich besser als die antistatischen Eigenschaften der Perückenhälfte, die mit der bekannten, stearinsäurefreien Zusammensetzung gemäss vorliegendem Vergleichsversuch behandelt wird.

Beispiel 2: Man verfährt wie in Beispiel 1 angegeben, setzt jedoch 1 Teil (an Stelle von 5 Teilen) Stearinsäure ein.

Das Shampoonieren und Konditionieren der Perücke im Halbseitentest mit obiger Lösung wird ebenfalls wie in Beispiel 1 durchgeführt, wobei die folgenden, in der nachstehenden Tabelle III angegebenen Kämmbarkeits-, Griff- und Schaumeigenschaftsnoten im Vergleich mit der Leerformulierung erzielt werden:

Tabelle III

| | nach der 1. Applikation | nach der 2. Applikation | nach der 3. Applikation |
|---|---|---|---|
| Nasskämmbarkeit | +2 | +(2-3) | +(2-3) |
| Trockenkämmbarkeit | +1 | +(1-2) | +(1-2) |
| Griff (nass) | +(0-1) | +1 | +1 |
| Griff (trocken) | +1 | +1 | +(1-2) |
| Schaumeigenschaften | +2 | +(2-3) | +(2-3) |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikaltionen beobachtet werden können.

Beispiel 3: Man verfährt wie in Beispiel 1 angegeben, stellt jedoch die Zusammensetzung mit der 10 %igen Zitronensäurelösung auf den pH-Wert von 6,86 (statt 5,0) ein.

Das Shampoonieren und Konditionieren der Perücke im Halbseitentest mit obiger Lösung wird ebenfalls wie in Beispiel 1 durchgeführt,

wobei die folgenden, in der nachstehenden Tabelle IV angegebenen Kämmbarkeits-, Griff- und Schaumeigenschaftsnoten im Vergleich mit der Leerformulierung erzielt werden:

Tabelle IV

| | nach der 1. Applikation | nach der 2. Applikation | nach der 3. Applikation |
|---|---|---|---|
| Nasskämmbarkeit | +2 | +(2-3) | +(2-3) |
| Trockenkämmbarkeit | +1 | +(1-2) | +(1-2) |
| Griff (nass) | +1 | +1 | +(1-2) |
| Griff (trocken) | +1 | +1 | +1 |
| Schaumeigenschaften | +2 | +2 | +2 |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

Beispiel 4: Man verfährt wie in Beispiel 1 angegeben, setzt jedoch 1 Teil (an Stelle von 5 Teilen) Stearinsäure und stellt die Zusammensetzung mit der 10 %igen Zitronensäurelösung auf den pH-Wert von 6,95 (statt 5,0) ein.

Das Shampoonieren und Konditionieren der Perücke im Halbseitentest mit obiger Lösung wird ebenfalls wie in Beispiel 1 durchgeführt, wobei die folgenden, in der nachstehenden Tabelle V angegebenen Kämmbarkeits-, Griff- und Schaumeigenschaftsnoten im Vergleich mit der Leerformulierung erzielt werden:

Tabelle V

| | nach der 1. Applikation | nach der 2. Applikation | nach der 3. Applikation |
|---|---|---|---|
| Nasskämmbarkeit | +(1-2) | +2 | +2 |
| Trockenkämmbarkeit | 0 | +1 | +(1-2) |
| Griff (nass) | 0 | +1 | +1 |
| Griff (trocken) | +1 | +1 | +(1-2) |
| Schaumeigenschaften | +2 | +2 | +2 |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

Beispiel 5: Man verfährt wie in Beispiel 1 angegeben, setzt jedoch 5 Teile Oelsäure (an Stelle von Stearinsäure) ein.

Das Shampoonieren und Konditionieren der Perücke im Halbseitentest mit obiger Lösung wird ebenfalls wie in Beispiel 1 durchgeführt, wobei die folgenden, in der nachstehenden Tabelle VI angegebenen Kämmbarkeits-, Griff- und Schaumeigenschaftsnoten im Vergleich mit der Leerformulierung erzielt werden:

Tabelle VI

| | nach der 1. Applikation | nach der 2. Applikation | nach der 3. Applikation |
|---|---|---|---|
| Nasskämmbarkeit | +(2-3) | +3 | +3 |
| Trockenkämmbarkeit | +(1-2) | +(0-1) | +1 |
| Griff (nass) | +1 | +(1-2) | +(1-2) |
| Griff (trocken) | +1 | +1 | +1 |
| Schaumeigenschaften | +(1-2) | +1 | +(1-2) |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

Beispiel 6: Man verfährt wie in Beispiel 1 angegeben, setzt jedoch 5 Teile Oelsäure (an Stelle von Stearinsäure) und stellt die Zusammensetzung mit der 10 %igen Zitronensäurelösung auf den pH-Wert von 7,10 (statt 5,0) ein.

Das Shampoonieren und Konditionieren der Perücke im Halbseitentest mit obiger Lösung wird ebenfalls wie in Beispiel 1 durchgeführt, wobei die folgenden, in der nachstehenden Tabelle VII angegebenen Kämmbarkeits-, Griff- und Schaumeigenschaftsnoten im Vergleich mit der Leerformulierung erzielt werden:

Tabelle VII

| | nach der 1. Applikation | nach der 2. Applikation | nach der 3. Applikation |
|---|---|---|---|
| Nasskämmbarkeit | +(1-2) | +2 | +(2-3) |
| Trockenkämmbarkeit | 0 | +(1-2) | 0 |
| Griff (nass) | 0 | +1 | +2 |
| Griff (trocken) | 0 | +(0-1) | +(0-1) |
| Schaumeigenschaften | +1 | +1 | +(1-2) |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

Beispiel 7: Man verfährt wie in Beispiel 1 angegeben, setzt jedoch 5 Teile Palmitinsäure (an Stelle von Stearinsäure) und stellt die Zusammensetzung mit der 10 %igen Zitronensäurelösung auf den pH-Wert von 6,80 (statt 5,0) ein.

Das Shampoonieren und Konditionieren der Perücke im Halbseitentest mit obiger Lösung wird ebenfalls wie in Beispiel 1 durchgeführt, wobei die folgenden, in der nachstehenden Tabelle VIII angegebenen Kämmbarkeits-, Griff- und Schaumeigenschaftsnoten im Vergleich mit der Leerformulierung erzielt werden:

Tabelle VIII

| | nach der 1. Applikation | nach der 2. Applikation | nach der 3. Applikation |
|---|---|---|---|
| Nasskämmbarkeit | +2 | +(2-3) | +3 |
| Trockenkämmbarkeit | +1 | +(1-2) | +1 |
| Griff (nass) | 0 | +(1-2) | +(1-2) |
| Griff (trocken) | +1 | +1 | +(1-2) |
| Schaumeigenschaften | +(2-3) | +(2-3) | +(2-3) |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

Beispiel 8: Man verfährt wie in Beispiel 1 angegeben, setzt jedoch 1 Teil Palmitinsäure (an Stelle von 5 Teilen Stearinsäure) und stellt die Zusammensetzung mit der 10 %igen Zitronensäurelösung auf den pH-Wert von 6,86 (statt 5,0) ein.

Das Shampoonieren und Konditionieren der Perücke im Halbseitentest mit obiger Lösung wird ebenfalls wie in Beispiel 1 durchgeführt, wobei die folgenden, in der nachstehenden Tabelle IX angegebenen Kämmbarkeits-, Griff- und Schaumeigenschaftsnoten im Vergleich mit der Leerformulierung erzielt werden:

Tabelle IX

| | nach der 1. Applikation | nach der 2. Applikation | nach der 3. Applikation |
|---|---|---|---|
| Nasskämmbarkeit | +2 | +(2-3) | +(2-3) |
| Trockenkämmbarkeit | 0 | 0 | +1 |
| Griff (nass) | 0 | +(1-2) | +(1-2) |
| Griff (trocken) | +1 | +1 | +1 |
| Schaumeigenschaften | +2 | +(2-3) | +(2-3) |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

Patentansprüche

1. Wässrige Zusammensetzung aus polymeren quaternären Ammoniumsalzen, Tensiden und Fettsäuren, dadurch gekennzeichnet, dass sie einen pH-Wert von höchstens 7,1 aufweist und mindestens

(A) quaternäre Copolymerisate aus Vinylpyrrolidon, Estern der Acryl- oder Methacrylsäure und Alkylaminoalkanolen, Poly(dialkyl- alkenylen-ammoniumhalogenid)-α,ω-(trialkanolammoniumhalogenid)-Verbindungen, Copolymerisate aus Dialkyldialkenylenammoniumsalzen und Acrylamid oder Methacrylamid, Copolymerisate aus Acryl- oder Methacrylsäure, -amid oder -nitril und quaternären Ammoniumsalzen der Acrylsäurereihe, quanternäre Copolymerisate auf Basis von Polyamid-Aminen oder, sofern nicht-ionische Tenside oder solche mit intramolekular je einer positiven und negativen Ladung als Komponenten ($B_2$) und ($B_3$) mindestens teilweise eingesetzt werden, auch Homopolymerisate aus Dialkyldialkenylenammoniumsalzen, Homopolymerisate aus quaternären Ammoniumsalzen der Acrylsäurereihe oder Homopolymerisate aus Vinyl-N-alkyl- oder -N-alkenylpyridiniumsalzen enthält, wobei die Komponente (A) in wässrigen Tensidsystemen löslich oder mikroemulgierbar ist und eine Molekulargewichtsverteilung von $10^3$ bis $10^9$ aufweist,

($B_1$) ein anionisches Tensid,

($B_2$) ein nicht-ionisches Tensid aus der Gruppe der Fettsäureester von 3- bis 6-wertigen Alkoholen oder von Mono- oder Disacchariden, der alkoxylierten Fettamine, Fettalkohole, Fettsäuren, Fettsäureamide, Alkylphenole oder Kohlenhydrate oder der Addukte aus Aethylen- und Propylenoxyd,

($B_3$) ein Tensid mit intramolekular je einer positiven und negativen Ladung
oder
($B_4$) ein Tensid mit intramolekular einer positiven und zwei negativen Ladungen

wobei die Tenside ($B_3$) und ($B_4$) als Gemisch mit mindestens einem der Tenside ($B_1$) und ($B_2$) eingesetzt werden, und

(C) eine Fettsäure mit 12 bis 22 Kohlenstoffatomen

enthält, wobei bei Einsatz eines anionischen Tensids und gegebenenfalls eines Tensides mit intramolekular einer positiven und zwei negativen Ladungen als Komponenten ($B_1$) und ($B_4$) das Tensid mindestens teilweise mit der Komponente (A) unter Ionenaustausch umgesetzt ist.

2. Zusammensetzung nach Anspruch 1, dadurch gekennzeichnet, dass sie als Komponente (A) mindestens

ein Copolymerisat aus Vinylpyrrolidon und Acrylaten oder Methacrylaten eines Molekulargewichtes von $1{,}5 \cdot 10^4$ bis $1 \cdot 10^6$, welches in beliebiger Reihenfolge durchschnittlich 20 bis 99 Mol-% wiederkehrende Strukturelemente der Formel

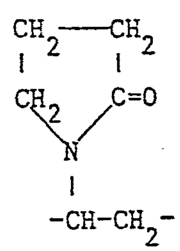

und durchschnittlich 1 bis 80 Mol% wiederkehrende Strukturelemente der Formel

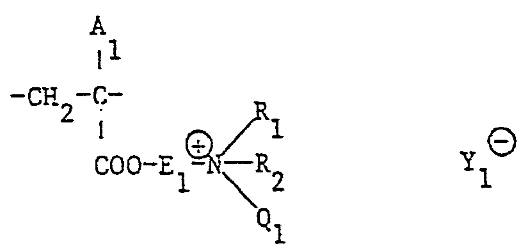

aufweist, worin $A_1$ Wasserstoff oder Methyl, $E_1$ gegebenenfalls durch Hydroxyl substituiertes Alkylen mit 2 bis 18 Kohlenstoffatomen, $R_1$ und $R_2$ je Alkyl mit 1 bis 4 Kohlenstoffatomen, $Q_1$ Wasserstoff, Methyl, Aethyl oder Benzyl und $Y_1^{\ominus}$ ein Sulfat- oder Halogenidanion, ein Alkylsulfat- oder Alkylphosphation oder das Anion einer Alkylcarbonsäure mit je 1 bis 4 Kohlenstoffatomen im Alkylrest,

ein Poly(dimethylbutenylammoniumchlorid)-α,ω-bis(triäthanolammoniumchlorid) eines Molekulargewichtes von 1000 bis 5000, welches der Formel

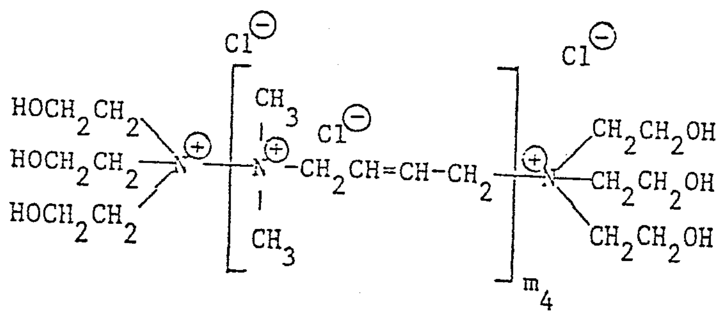

entspricht, worin $m_4$ eine ganze Zahl von 4 bis 35 ist,

ein Copolymerisat aus Dimethyldiallylammoniumchlorid und Acrylamid eines Molekulargewichtes von $5 \cdot 10^3$ bis $5 \cdot 10^6$, welches in beliebiger Reihenfolge durchschnittlich 20 bis 99 Mol-% wiederkehrende Strukturelemente der Formel

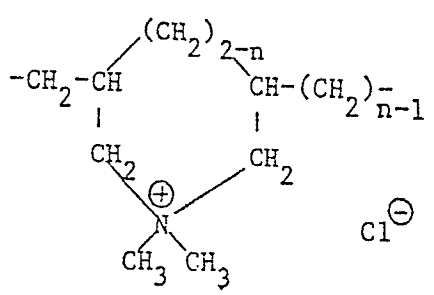

,

worin n 1 oder 2 ist, und durchschnittlich 1 bis 80 Mol% wiederkehrende Strukturelemente der Formel

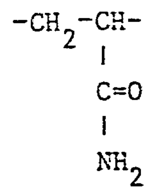

aufweist,

ein Copolymerisat aus Verbindungen der Acrylsäurereihe und quaternären Ammoniumsalzen der Acrylsäurereihe einer Molekulargewichtsverteilung von $10^4$ bis $10^9$, welches in beliebiger Reihenfolge wiederkehrende Strukturelemente der Formeln

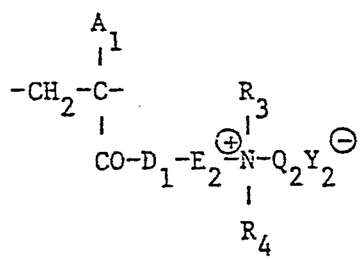 ,

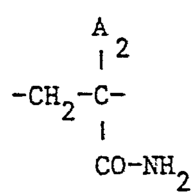

und gegebenenfalls

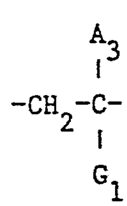 und 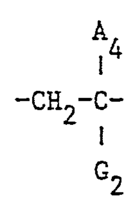 .

aufweist, worin $A_1$, $A_2$, $A_3$ und $A_4$ je Wasserstoff oder Methyl, $G_1$ und $G_2$ voneinander verschieden sind und je -CN, -COOH oder

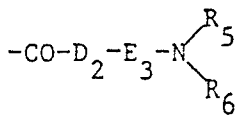 , $D_1$ und $D_2$ je Sauerstoff oder -NH-, $E_2$ und $E_3$ je Alkylen mit 1 bis 4 Kohlenstoffatomen, das gegebenenfalls durch Hydroxyl substituiert ist, $R_3$, $R_4$, $R_5$ und $R_6$ je Methyl oder Aethyl, $Q_2$ Alkyl, Hydroxyalkyl mit 1 bis 4 Kohlenstoffatomen oder Benzyl und $Y_2^{\ominus}$ ein Sulfat- oder Halogenidanion oder ein Alkylsulfat- oder Alkylphosphation mit 1 bis 4 Kohlenstoffatomen im Alkylrest bedeuten,

ein Copolymerisat aus aliphatischen, gesättigten Dicarbonsäuren und Polyalkylenpolyaminen eines Molekulargewichtes von $10^3$ bis $10^5$ welches in beliebiger Reihenfolge wiederkehrende Strukturelemente der Formeln

$$-CO-(CH_2)_4-CO-$$

und

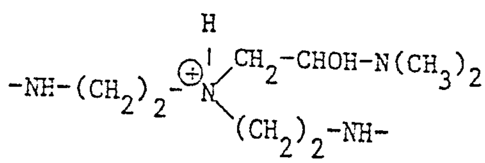

aufweist,

ein Homopolymerisat aus Dimethyldiallylammoniumchlorid eines Molekulargewichtes von 4000 bis 550000, welches wiederkehrende Strukturelemente der Formel

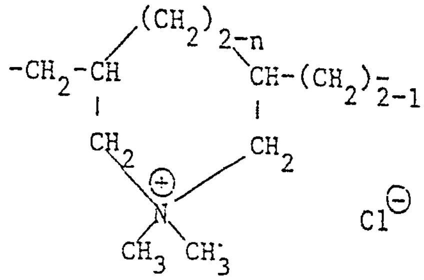 ,

aufweist, worin n 1 oder 2 ist,

ein Homopolymerisat aus quaternären Ammoniumsalzen der Acrylsäurereihe einer Molekulargewichtsverteilung von $10^4$ bis $10^9$, welches wiederkehrende Strukturelemente der Formel

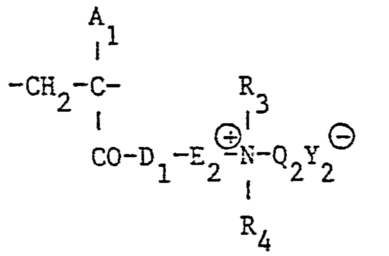

,

aufweist, worin $A_1$, $D_1$, $E_2$, $R_3$, $R_4$, $Q_2$ und $Y_2^{\ominus}$ die angegebenen Bedeutungen haben, oder

ein Homopolymerisat aus Vinylpyridiniumsalzen eines Molekulargewichtes von $5 \cdot 10^3$ bis $1 \cdot 10^5$, welches wiederkehrende Strukturelemente der Formel

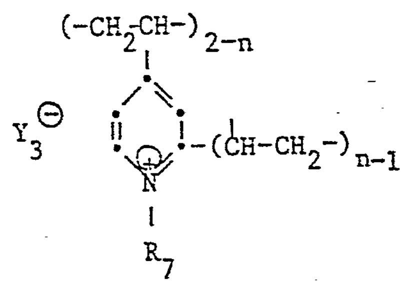

aufweist, worin n 1 oder 2, $R_7$ Alkyl mit 1 bis 22 Kohlenstoffatomen oder Alkenyl mit 3 oder 4 Kohlenstoffatomen und $Y_3^{\ominus}$ ein Halogenid- oder Sulfat-Anion oder das Anion einer Alkylcarbonsäure mit 1 bis 4 Kohlenstoffatomen im Alkylrest bedeuten,

enthält.

3. Zusammensetzung nach Anspruch 2, dadurch gekennzeichnet, dass sie als Komponente (A) ein Copolymer einer Molekulargewichtsverteilung von $10^4$ bis $10^9$ enthält, wobei das Molekulargewicht von mindestens 5 Gewichtsprozent des copolymeren Ammoniumsalzes $10^7$ bis $10^9$ beträgt, und das Copolymer in beliebiger Reihenfolge durchschnittlich 1 bis 90 Mol-% wiederkehrende Strukturelemente der Formel

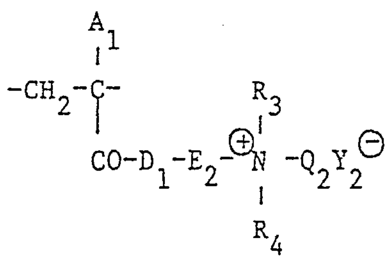 ,

durchschnittlich 10 bis 99 Mol-% wiederkehrende Strukturelemente der Formel

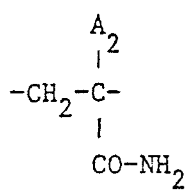 und

jeweils 0 bis durchschnittlich 10 Mol-% wiederkehrende Strukturelemente der Formel

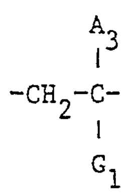

und gegebenenfalls

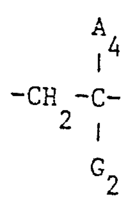

aufweist, worin $A_1$, $A_2$, $A_3$, $A_4$, $D_1$, $E_2$, $G_1$, $G_2$, $R_3$, $R_4$, $Q_2$ und $Y_2^{\ominus}$ jeweils die in Anspruch 2 angegebenen Bedeutungen haben.

4. Zusammensetzung nach Anspruch 1, dadurch gekennzeichnet, dass sie als anionisches Tensid für die Komponente ($B_1$) ein Tensid der Formel $X_1^{\ominus} Z^{\oplus}$ enthält, worin $X_1^{\ominus}$ den Rest eines oberflächenaktiven Sarkosinats, Sulfats (Sulfatäthers), Sulfonats oder Sulfobernsteinsäurederivates, eines fluorierten Tensids oder eines Phosphattensids und $Z^{\oplus}$ ein Alkalimetall- oder ein gegebenenfalls durch 1 bis 3 ($C_1$-$C_4$)-Alkyl- oder -Alkanolreste substituiertes Ammoniumkation bedeuten.

5. Zusammensetzung nach Anspruch 1, dadurch gekennzeichnet, dass sie als Tensid mit intramolekular je einer positiven und negativen Ladung für die Komponente ($B_3$) Betaine oder Sulfobetaine enthält, die sich von einem Imidazolinderivat oder einem offenkettigen, aliphatischen Amin ableiten.

6. Zusammensetzung nach Anspruch 1, dadurch gekennzeichnet, dass sie als Tensid mit intramolekular einer positiven und zwei negativen Ladungen für die Komponente ($B_4$) ein Tensid der Formel $X_2^{\ominus} Z^{\oplus}$ enthält, worin $X_2^{\ominus}$ den Rest eines oberflächenaktiven N-alkyl-α-iminodipropionats oder eines in 2-Stellung alkylsubstituierten Imidazolinium-dicarbonsäurederivats bedeutet und $Z^{\oplus}$ die in Anspruch 4 angegebenen Bedeutungen hat.

7. Verfahren zur Herstellung der Zusammensetzung gemäss einem der Ansprüche 1 bis 6, dadurch gekennzeichnet, dass man die Komponente (A) mit mindestens einem Tensid als Komponenten ($B_1$) bis ($B_4$) und

mindestens einer Fettsäure als Komponente (C) bei 10 bis 90° C und einem pH-Wert von höchstens 7,1 vermischt, wobei man gleichzeitig, sofern ein anionisches Tensid als Komponente ($B_1$) allein oder in Gemisch mit einem Tensid mit intramolekular einer positiven und zwei negativen Ladungen als Komponente ($B_4$) eingesetzt wird, das Tensid mit der Komponente (A) unter Ionenaustausch mindestens teilweise umsetzt.

8. Verwendung der Zusammensetzung gemäss einem der Ansprüche 1 bis 6 in kosmetischen Mitteln.

9. Kosmetische Mittel, dadurch gekennzeichnet, dass sie mindestens eine der Zusammensetzungen gemäss einem der Ansprüche 1 bis 6 enthalten.

10. Haarbehandlungsverfahren, dadurch gekennzeichnet, dass man das Mittel gemäss Anspruch 9 auf mit Leitungswasser angefeuchtetem Haar bei 20 bis 40°C aufbringt und das Haar shampooniert und konditioniert.

FO 7.1/PLP/jt*